# EUROPEAN PATENT APPLICATION

(11) **EP 2 682 479 A1**
(43) Date of publication of application: **08.01.2014**
(21) Application number: 12753027.7
(22) Date of filing: 28.02.2012
(51) Int. Cl.: C12Q 1/68

(54) **METHOD FOR DNA AMPLIFICATION BASED ON THE ORIGINS OF REPLICATION OF THE BACTERIOPHAGE phi29 AND ASSOCIATED NUCLEOTIDE SEQUENCES**

(30) Priority: 03.03.2011 ES 201130288 P
(71) Applicant: Consejo Superior De Investigaciones Científicas (CSIC), 28006 Madrid (ES)
(72) Inventor: SALAS FALGUERAS, Margarita, E-28049 Madrid (ES); MENCÍA CABALLERO, Mario, E-28049 Madrid (ES); DE VEGA JOSÉ, Miguel, E-28049 Madrid (ES); LÁZARO BOLOS, José María, E-28049 Madrid (ES); GELLA MONTERO, Pablo, E-28049 Madrid (ES)
(74) Representative: Lahrtz, Fritz
(86) International application number: PCT/ES2012/070121
(87) International publication number: WO 2012/117141

(57) **Abstract**

The present invention refers to a DNA amplification method based on the origins of replication of bacteriophage ϕ29, and to the genic constructs, vectors and oligonucleotides that can be used in the method for amplifying an exogenous sequence of interest.

SEQUENCE LISTING

<110> Consejo Superior de Investigaciones Cientificas (CSIC)

<120> METHOD FOR DNA AMPLIFICATION BASED ON THE ORIGINS OF REPLICATION
OF THE BACTERIOPHAGE 29 AND ASSOCIATED NUCLEOTIDE SEQUENCES

<130> BL68755PCEP

<140> EP 12753027.7
<141> 2012-02-28

<150> ES P201130288
<151> 2011-03-03

<160> 15

<170> PatentIn version 3.5

<210> 1
<211> 5833
<212> DNA
<213> Artificial Sequence

<220>
<223> Vector pETORPHI.

<400> 1
atccggatat agttcctcct ttcagcaaaa aacccctcaa gacccgttta gaggccccaa 60

ggggttatgc tagttattgc tcagcggtgg cagcagccaa ctcagcttcc tttcgggctt 120

tgttagcagc cggatctcag tggtggtggt ggtggtgctc gagtgcggcc gcaagcttgc 180

atgcaggcct ctgcagtcga cgggcccggg atccgatcca atcagtggct gtgcgacaca 240

gacgaagcgc taaaacgtgg gattctgtgt cgttttatgt tgttcattga caaacctatc 300

tagtaggtct atagattgat atattaagta gtagtttccc tccctgatca tggtatgccg 360

aggatcgacg gattatgtcg atattaggag aatggtatca tgtgagggtg ggggcttact 420

ttaaagtagg gtacagcgac aacatacacc atttccccat tgaccgacta tcttcgacaa 480

gaatctaaca actaaatcac gactatatac ctatactatt tattatcatc aatttgtcga 540

aaagggtaga caaactatcg tttaacatgt tatactataa tagaagtaag gtaataagac 600

aaccaatcat aggaggcacg agattgatct agatgcattc gcgaggtacc gagctcgaat 660

tcggatcccg acccatttgc tgtccaccag tcatgctagc catatggctg ccgcgcggca 720

ccaggccgct gctgtgatga tgatgatgat ggctgctgcc catggtatat ctccttctta 780

aagttaaaca aaattatttc tagaggggaa ttgttatccg ctcacaattc ccctatagtg 840

agtcgtatta atttcgcggg atcgagatct cgatcctcta cgccggacgc atcgtggccg 900

gcatcaccgg cgccacaggt gcggttgctg gcgcctatat cgccgacatc accgatgggg 960

aagatcgggc tcgccacttc gggctcatga gcgcttgttt cggcgtgggt atggtggcag 1020

gccccgtggc cgggggactg ttgggcgcca tctccttgca tgcaccattc cttgcggcgg 1080

cggtgctcaa cggcctcaac ctactactgg gctgcttcct aatgcaggag tcgcataagg 1140

gagagcgtcg agatcccgga caccatcgaa tggcgcaaaa cctttcgcgg tatggcatga 1200

tagcgcccgg aagagagtca attcagggtg gtgaatgtga aaccagtaac gttatacgat 1260

gtcgcagagt atgccggtgt ctcttatcag accgtttccc gcgtggtgaa ccaggccagc 1320

cacgtttctg cgaaaacgcg ggaaaaagtg gaagcggcga tggcggagct gaattacatt 1380

cccaaccgcg tggcacaaca actggcgggc aaacagtcgt tgctgattgg cgttgccacc 1440

tccagtctgg ccctgcacgc gccgtcgcaa attgtcgcgg cgattaaatc tcgcgccgat 1500

caactgggtg ccagcgtggt ggtgtcgatg gtagaacgaa gcggcgtcga agcctgtaaa 1560

gcggcggtgc acaatcttct cgcgcaacgc gtcagtgggc tgatcattaa ctatccgctg 1620

gatgaccagg atgccattgc tgtggaagct gcctgcacta atgttccggc gttatttctt 1680

gatgtctctg accagacacc catcaacagt attattttct cccatgaaga cggtacgcga 1740

ctgggcgtgg agcatctggt cgcattgggt caccagcaaa tcgcgctgtt agcgggccca 1800

ttaagttctg tctcggcgcg tctgcgtctg gctggctggc ataaatatct cactcgcaat 1860

caaattcagc cgatagcgga acgggaaggc gactggagtg ccatgtccgg ttttcaacaa 1920

accatgcaaa tgctgaatga gggcatcgtt cccactgcga tgctggttgc caacgatcag 1980

atggcgctgg gcgcaatgcg cgccattacc gagtccgggc tgcgcgttgg tgcggatatc 2040

tcggtagtgg gatacgacga taccgaagac agctcatgtt atatcccgcc gttaaccacc 2100

atcaaacagg attttcgcct gctggggcaa accagcgtgg accgcttgct gcaactctct 2160

cagggccagg cggtgaaggg caatcagctg ttgcccgtct cactggtgaa aagaaaaacc 2220

accctggcgc ccaatacgca aaccgcctct ccccgcgcgt tggccgattc attaatgcag 2280

ctggcacgac aggtttcccg actggaaagc gggcagtgag cgcaacgcaa ttaatgtaag 2340

ttagctcact cattaggcac cgggatctcg accgatgccc ttgagagcct tcaacccagt 2400

cagctccttc cggtgggcgc ggggcatgac tatcgtcgcc gcacttatga ctgtcttctt 2460

tatcatgcaa ctcgtaggac aggtgccggc agcgctctgg gtcattttcg gcgaggaccg 2520

ctttcgctgg agcgcgacga tgatcggcct gtcgcttgcg gtattcggaa tcttgcacgc 2580

cctcgctcaa gccttcgtca ctggtcccgc caccaaacgt ttcggcgaga agcaggccat 2640

tatcgccggc atggcggccc cacgggtgcg catgatcgtg ctcctgtcgt tgaggacccg 2700

gctaggctgg cggggttgcc ttactggtta gcagaatgaa tcaccgatac gcgagcgaac 2760

gtgaagcgac tgctgctgca aaacgtctgc gacctgagca acaacatgaa tggtcttcgg 2820

tttccgtgtt tcgtaaagtc tggaaacgcg gaagtcagcg ccctgcacca ttatgttccg 2880

gatctgcatc gcaggatgct gctggctacc ctgtggaaca cctacatctg tattaacgaa 2940

gcgctggcat tgaccctgag tgatttttct ctggtcccgc cgcatccata ccgccagttg 3000

tttaccctca caacgttcca gtaaccgggc atgttcatca tcagtaaccc gtatcgtgag 3060

catcctctct cgtttcatcg gtatcattac ccccatgaac agaaatcccc cttacacgga 3120

ggcatcagtg accaaacagg aaaaaaccgc ccttaacatg gcccgcttta tcagaagcca 3180

gacattaacg cttctggaga aactcaacga gctggacgcg gatgaacagg cagacatctg 3240

tgaatcgctt cacgaccacg ctgatgagct ttaccgcagc tgcctcgcgc gtttcggtga 3300

tgacggtgaa aacctctgac acatgcagct cccggagacg gtcacagctt gtctgtaagc 3360

ggatgccggg agcagacaag cccgtcaggg cgcgtcagcg ggtgttggcg ggtgtcgggg 3420

cgcagccatg acccagtcac gtagcgatag cggagtgtat actggcttaa ctatgcggca 3480

tcagagcaga ttgtactgag agtgcaccat atatgcggtg tgaaataccg cacagatgcg 3540

taaggagaaa ataccgcatc aggcgctctt ccgcttcctc gctcactgac tcgctgcgct 3600

cggtcgttcg gctgcggcga gcggtatcag ctcactcaaa ggcggtaata cggttatcca 3660

cagaatcagg ggataacgca ggaaagaaca tgtgagcaaa aggccagcaa aaggccagga 3720

accgtaaaaa ggccgcgttg ctggcgtttt tccataggct ccgcccccct gacgagcatc 3780

acaaaaatcg acgctcaagt cagaggtggc gaaacccgac aggactataa agataccagg 3840

cgtttccccc tggaagctcc ctcgtgcgct ctcctgttcc gaccctgccg cttaccggat 3900

acctgtccgc ctttctccct tcgggaagcg tggcgctttc tcatagctca cgctgtaggt 3960

atctcagttc ggtgtaggtc gttcgctcca agctgggctg tgtgcacgaa ccccccgttc 4020

agcccgaccg ctgcgcctta tccggtaact atcgtcttga gtccaacccg gtaagacacg 4080

acttatcgcc actggcagca gccactggta acaggattag cagagcgagg tatgtaggcg 4140

gtgctacaga gttcttgaag tggtggccta actacggcta cactagaagg acagtatttg 4200

gtatctgcgc tctgctgaag ccagttacct tcggaaaaag agttggtagc tcttgatccg 4260

gcaaacaaac caccgctggt agcggtggtt tttttgtttg caagcagcag attacgcgca 4320

gaaaaaaagg atctcaagaa gatcctttga tcttttctac ggggtctgac gctcagtgga 4380

acgaaaactc acgttaaggg attttggtca tgaacaataa aactgtctgc ttacataaac 4440

agtaatacaa ggggtgttat gagccatatt caacgggaaa cgtcttgctc taggccgcga 4500

ttaaattcca acatggatgc tgatttatat gggtataaat gggctcgcga taatgtcggg 4560

caatcaggtg cgacaatcta tcgattgtat gggaagcccg atgcgccaga gttgtttctg 4620

aaacatggca aaggtagcgt tgccaatgat gttacagatg agatggtcag actaaactgg 4680

ctgacggaat ttatgcctct tccgaccatc aagcatttta tccgtactcc tgatgatgca 4740

tggttactca ccactgcgat ccccgggaaa acagcattcc aggtattaga agaatatcct 4800

gattcaggtg aaaatattgt tgatgcgctg gcagtgttcc tgcgccggtt gcattcgatt 4860

cctgtttgta attgtccttt taacagcgat cgcgtatttc gtctcgctca ggcgcaatca 4920

cgaatgaata acggtttggt tgatgcgagt gattttgatg acgagcgtaa tggctggcct 4980

gttgaacaag tctggaaaga aatgcataaa cttttgccat tctcaccgga ttcagtcgtc 5040

actcatggtg atttctcact tgataacctt atttttgacg aggggaaatt aataggttgt 5100

attgatgttg gacgagtcgg aatcgcagac cgataccagg atcttgccat cctatggaac 5160

tgcctcggtg agttttctcc ttcattacag aaacggcttt ttcaaaaata tggtattgat 5220

aatcctgata tgaataaatt gcagtttcat ttgatgctcg atgagttttt ctaagaatta 5280

attcatgagc ggatacatat ttgaatgtat ttagaaaaat aaacaaatag gggttccgcg 5340

cacatttccc cgaaaagtgc cacctgaaat tgtaaacgtt aatattttgt taaaattcgc 5400

gttaaatttt tgttaaatca gctcattttt taaccaatag gccgaaatcg gcaaaatccc 5460

ttataaatca aaagaataga ccgagatagg gttgagtgtt gttccagttt ggaacaagag 5520

tccactatta aagaacgtgg actccaacgt caaagggcga aaaaccgtct atcagggcga 5580

tggcccacta cgtgaaccat caccctaatc aagttttttg gggtcgaggt gccgtaaagc 5640

actaaatcgg aaccctaaag ggagcccccg atttagagct tgacggggaa agccggcgaa 5700

cgtggcgaga aaggaaggga agaaagcgaa aggagcgggc gctagggcgc tggcaagtgt 5760

agcggtcacg ctgcgcgtaa ccaccacacc cgccgcgctt aatgcgccgc tacagggcgc 5820

gtcccattcg cca 5833

<210> 2
<211> 5666
<212> DNA
<213> Artificial Sequence

<220>
<223> Vector pETORPHI68L.

<400> 2
atccggatat agttcctcct ttcagcaaaa aacccctcaa gacccgttta gaggccccaa 60

ggggttatgc tagttattgc tcagcggtgg cagcagccaa ctcagcttcc tttcgggctt 120

tgttagcagc cggatctcag tggtggtggt ggtggtgctc gagtgcggcc gcaagcttgc 180

atgcaggcct ctgcagtcga cgggcccggg atccgatcca atcagtggct gtgcgacaca 240

gacgaagcgc taaaacgtgg gattctgtgt cgttttatgt tgttcattga caaacctatc 300

tagtaggtct atagattgat atattaagta gtagtttccc tccctgatca tggtatgccg 360

aggatcgacg gattatgtcg atattaggag aatggtatca tgtgagggtg ggggcttact 420

ttaaagtaag cccccaccct cacatgatac cattctccta atatcgacat aatccgtcga 480

tcctcggcat aattcggatc ccgacccatt tgctgtccac cagtcatgct agccatatgg 540

ctgccgcgcg gcaccaggcc gctgctgtga tgatgatgat gatggctgct gcccatggta 600

tatctccttc ttaaagttaa acaaaattat ttctagaggg gaattgttat ccgctcacaa 660

ttcccctata gtgagtcgta ttaatttcgc gggatcgaga tctcgatcct ctacgccgga 720

cgcatcgtgg ccggcatcac cggcgccaca ggtgcggttg ctggcgccta tatcgccgac 780

atcaccgatg gggaagatcg ggctcgccac ttcgggctca tgagcgcttg tttcggcgtg 840

ggtatggtgg caggccccgt ggccggggga ctgttgggcg ccatctcctt gcatgcacca 900

ttccttgcgg cggcggtgct caacggcctc aacctactac tgggctgctt cctaatgcag 960

gagtcgcata agggagagcg tcgagatccc ggacaccatc gaatggcgca aaacctttcg 1020

cggtatggca tgatagcgcc cggaagagag tcaattcagg gtggtgaatg tgaaaccagt 1080

aacgttatac gatgtcgcag agtatgccgg tgtctcttat cagaccgttt cccgcgtggt 1140

gaaccaggcc agccacgttt ctgcgaaaac gcgggaaaaa gtggaagcgg cgatggcgga 1200

gctgaattac attcccaacc gcgtggcaca acaactggcg ggcaaacagt cgttgctgat 1260

tggcgttgcc acctccagtc tggccctgca cgcgccgtcg caaattgtcg cggcgattaa 1320

atctcgcgcc gatcaactgg gtgccagcgt ggtggtgtcg atggtagaac gaagcggcgt 1380

cgaagcctgt aaagcggcgg tgcacaatct tctcgcgcaa cgcgtcagtg ggctgatcat 1440

taactatccg ctggatgacc aggatgccat tgctgtggaa gctgcctgca ctaatgttcc 1500

ggcgttattt cttgatgtct ctgaccagac acccatcaac agtattattt tctcccatga 1560

agacggtacg cgactgggcg tggagcatct ggtcgcattg ggtcaccagc aaatcgcgct 1620

gttagcgggc ccattaagtt ctgtctcggc gcgtctgcgt ctggctggct ggcataaata 1680

tctcactcgc aatcaaattc agccgatagc ggaacgggaa ggcgactgga gtgccatgtc 1740

cggttttcaa caaaccatgc aaatgctgaa tgagggcatc gttcccactg cgatgctggt 1800

tgccaacgat cagatggcgc tgggcgcaat gcgcgccatt accgagtccg ggctgcgcgt 1860

tggtgcggat atctcggtag tgggatacga cgataccgaa gacagctcat gttatatccc 1920

gccgttaacc accatcaaac aggattttcg cctgctgggg caaaccagcg tggaccgctt 1980

gctgcaactc tctcagggcc aggcggtgaa gggcaatcag ctgttgcccg tctcactggt 2040

gaaaagaaaa accaccctgg cgcccaatac gcaaaccgcc tctccccgcg cgttggccga 2100

ttcattaatg cagctggcac gacaggtttc ccgactggaa agcgggcagt gagcgcaacg 2160

caattaatgt aagttagctc actcattagg caccgggatc tcgaccgatg cccttgagag 2220

ccttcaaccc agtcagctcc ttccggtggg cgcggggcat gactatcgtc gccgcactta 2280

tgactgtctt ctttatcatg caactcgtag gacaggtgcc ggcagcgctc tgggtcattt 2340

tcggcgagga ccgctttcgc tggagcgcga cgatgatcgg cctgtcgctt gcggtattcg 2400

gaatcttgca cgccctcgct caagccttcg tcactggtcc cgccaccaaa cgtttcggcg 2460

agaagcaggc cattatcgcc ggcatggcgg ccccacgggt gcgcatgatc gtgctcctgt 2520

cgttgaggac ccggctaggc tggcggggtt gccttactgg ttagcagaat gaatcaccga 2580

tacgcgagcg aacgtgaagc gactgctgct gcaaaacgtc tgcgacctga gcaacaacat 2640

gaatggtctt cggtttccgt gtttcgtaaa gtctggaaac gcggaagtca gcgccctgca 2700

ccattatgtt ccggatctgc atcgcaggat gctgctggct accctgtgga acacctacat 2760

ctgtattaac gaagcgctgg cattgaccct gagtgatttt tctctggtcc cgccgcatcc 2820

ataccgccag ttgtttaccc tcacaacgtt ccagtaaccg ggcatgttca tcatcagtaa 2880

cccgtatcgt gagcatcctc tctcgtttca tcggtatcat tacccccatg aacagaaatc 2940

ccccttacac ggaggcatca gtgaccaaac aggaaaaaac cgcccttaac atggcccgct 3000

ttatcagaag ccagacatta acgcttctgg agaaactcaa cgagctggac gcggatgaac 3060

aggcagacat ctgtgaatcg cttcacgacc acgctgatga gctttaccgc agctgcctcg 3120

cgcgtttcgg tgatgacggt gaaaacctct gacacatgca gctcccggag acggtcacag 3180

cttgtctgta agcggatgcc gggagcagac aagcccgtca gggcgcgtca gcgggtgttg 3240

gcgggtgtcg gggcgcagcc atgacccagt cacgtagcga tagcggagtg tatactggct 3300

taactatgcg gcatcagagc agattgtact gagagtgcac catatatgcg gtgtgaaata 3360

ccgcacagat gcgtaaggag aaaataccgc atcaggcgct cttccgcttc ctcgctcact 3420

gactcgctgc gctcggtcgt tcggctgcgg cgagcggtat cagctcactc aaaggcggta 3480

atacggttat ccacagaatc aggggataac gcaggaaaga acatgtgagc aaaaggccag 3540

caaaaggcca ggaaccgtaa aaaggccgcg ttgctggcgt ttttccatag gctccgcccc 3600

cctgacgagc atcacaaaaa tcgacgctca agtcagaggt ggcgaaaccc gacaggacta 3660

taaagatacc aggcgtttcc ccctggaagc tccctcgtgc gctctcctgt tccgaccctg 3720

ccgcttaccg gatacctgtc cgcctttctc ccttcgggaa gcgtggcgct ttctcatagc 3780

tcacgctgta ggtatctcag ttcggtgtag gtcgttcgct ccaagctggg ctgtgtgcac 3840

gaaccccccg ttcagcccga ccgctgcgcc ttatccggta actatcgtct tgagtccaac 3900

ccggtaagac acgacttatc gccactggca gcagccactg gtaacaggat tagcagagcg 3960

aggtatgtag gcggtgctac agagttcttg aagtggtggc ctaactacgg ctacactaga 4020

aggacagtat ttggtatctg cgctctgctg aagccagtta ccttcggaaa aagagttggt 4080

agctcttgat ccggcaaaca aaccaccgct ggtagcggtg gtttttttgt ttgcaagcag 4140

cagattacgc gcagaaaaaa aggatctcaa gaagatcctt tgatcttttc tacggggtct 4200

gacgctcagt ggaacgaaaa ctcacgttaa gggattttgg tcatgaacaa taaaactgtc 4260

tgcttacata aacagtaata caaggggtgt tatgagccat attcaacggg aaacgtcttg 4320

ctctaggccg cgattaaatt ccaacatgga tgctgattta tatgggtata aatgggctcg 4380

cgataatgtc gggcaatcag gtgcgacaat ctatcgattg tatgggaagc ccgatgcgcc 4440

agagttgttt ctgaaacatg gcaaaggtag cgttgccaat gatgttacag atgagatggt 4500

cagactaaac tggctgacgg aatttatgcc tcttccgacc atcaagcatt ttatccgtac 4560

tcctgatgat gcatggttac tcaccactgc gatccccggg aaaacagcat tccaggtatt 4620

agaagaatat cctgattcag gtgaaaatat tgttgatgcg ctggcagtgt tcctgcgccg 4680

gttgcattcg attcctgttt gtaattgtcc ttttaacagc gatcgcgtat ttcgtctcgc 4740

tcaggcgcaa tcacgaatga ataacggttt ggttgatgcg agtgattttg atgacgagcg 4800

taatggctgg cctgttgaac aagtctggaa agaaatgcat aaacttttgc cattctcacc 4860

ggattcagtc gtcactcatg gtgatttctc acttgataac cttatttttg acgaggggaa 4920

attaataggt tgtattgatg ttggacgagt cggaatcgca gaccgatacc aggatcttgc 4980

catcctatgg aactgcctcg gtgagttttc tccttcatta cagaaacggc tttttcaaaa 5040

atatggtatt gataatcctg atatgaataa attgcagttt catttgatgc tcgatgagtt 5100

tttctaagaa ttaattcatg agcggataca tatttgaatg tatttagaaa aataaacaaa 5160

taggggttcc gcgcacattt ccccgaaaag tgccacctga aattgtaaac gttaatattt 5220

tgttaaaatt cgcgttaaat ttttgttaaa tcagctcatt ttttaaccaa taggccgaaa 5280

tcggcaaaat cccttataaa tcaaaagaat agaccgagat agggttgagt gttgttccag 5340

tttggaacaa gagtccacta ttaaagaacg tggactccaa cgtcaaaggg cgaaaaaccg 5400

tctatcaggg cgatggccca ctacgtgaac catcacccta atcaagtttt ttggggtcga 5460

ggtgccgtaa agcactaaat cggaacccta aagggagccc ccgatttaga gcttgacggg 5520

gaaagccggc gaacgtggcg agaaaggaag ggaagaaagc gaaaggagcg ggcgctaggg 5580

cgctggcaag tgtagcggtc acgctgcgcg taaccaccac acccgccgcg cttaatgcgc 5640

cgctacaggg cgcgtcccat tcgcca 5666

<210> 3
<211> 5871
<212> DNA
<213> Artificial Sequence

<220>
<223> Vector pETORPHIBae.

<400> 3
atccggatat agttcctcct ttcagcaaaa aacccctcaa gacccgttta gaggccccaa 60

ggggttatgc tagttattgc tcagcggtgg cagcagccaa ctcagcttcc tttcgggctt 120

tgttagcagc cggatctcag tggtggtggt ggtggtgctc gagtgcggcc gcaagcttgc 180

atgcaggcct ctgcagtcga cgggcccggg atccgatcca atcagtggct gtgcgacaca 240

gacgaagcgc taaaacgtgg gattctgtgt cgttttatgt tgttcattga caaacctatc 300

tagtaggtct atagattgat atattaagta gtagtttccc tccctgatca tggtatgccg 360

aggatcgacg gattatgtcg atattaggag aatggtatca tgtgagggtg ggggcttact 420

tttctcctaa ttatttgact gtcgtatctg tcatcaacca aaagtagggt acagcgacaa 480

catacaccat ttccccattg accgactatc ttcgacaaga atctaacaac taaatcacga 540

ctatatacct atactattta ttatcatcaa tttgtcgaaa agggtagaca aactatcgtt 600

taacatgtta tactataata gaagtaaggt aataagacaa ccaatcatag gaggcacgag 660

attgatctag atgcattcgc gaggtaccga gctcgaattc ggatcccgac ccatttgctg 720

tccaccagtc atgctagcca tatggctgcc gcgcggcacc aggccgctgc tgtgatgatg 780

atgatgatgg ctgctgccca tggtatatct ccttcttaaa gttaaacaaa attatttcta 840

gaggggaatt gttatccgct cacaattccc ctatagtgag tcgtattaat ttcgcgggat 900

cgagatctcg atcctctacg ccggacgcat cgtggccggc atcaccggcg ccacaggtgc 960

ggttgctggc gcctatatcg ccgacatcac cgatggggaa gatcgggctc gccacttcgg 1020

gctcatgagc gcttgtttcg gcgtgggtat ggtggcaggc cccgtggccg ggggactgtt 1080

gggcgccatc tccttgcatg caccattcct tgcggcggcg gtgctcaacg gcctcaacct 1140

actactgggc tgcttcctaa tgcaggagtc gcataaggga gagcgtcgag atcccggaca 1200

ccatcgaatg gcgcaaaacc tttcgcggta tggcatgata gcgcccggaa gagagtcaat 1260

tcagggtggt gaatgtgaaa ccagtaacgt tatacgatgt cgcagagtat gccggtgtct 1320

cttatcagac cgtttcccgc gtggtgaacc aggccagcca cgtttctgcg aaaacgcggg 1380

aaaaagtgga agcggcgatg gcggagctga attacattcc caaccgcgtg gcacaacaac 1440

tggcgggcaa acagtcgttg ctgattggcg ttgccacctc cagtctggcc ctgcacgcgc 1500

cgtcgcaaat tgtcgcggcg attaaatctc gcgccgatca actgggtgcc agcgtggtgg 1560

tgtcgatggt agaacgaagc ggcgtcgaag cctgtaaagc ggcggtgcac aatcttctcg 1620

cgcaacgcgt cagtgggctg atcattaact atccgctgga tgaccaggat gccattgctg 1680

tggaagctgc ctgcactaat gttccggcgt tatttcttga tgtctctgac cagacaccca 1740

tcaacagtat tattttctcc catgaagacg gtacgcgact gggcgtggag catctggtcg 1800

cattgggtca ccagcaaatc gcgctgttag cgggcccatt aagttctgtc tcggcgcgtc 1860

tgcgtctggc tggctggcat aaatatctca ctcgcaatca aattcagccg atagcggaac 1920

gggaaggcga ctggagtgcc atgtccggtt ttcaacaaac catgcaaatg ctgaatgagg 1980

gcatcgttcc cactgcgatg ctggttgcca acgatcagat ggcgctgggc gcaatgcgcg 2040

ccattaccga gtccgggctg cgcgttggtg cggatatctc ggtagtggga tacgacgata 2100

ccgaagacag ctcatgttat atcccgccgt taaccaccat caaacaggat tttcgcctgc 2160

tggggcaaac cagcgtggac cgcttgctgc aactctctca gggccaggcg gtgaagggca 2220

atcagctgtt gcccgtctca ctggtgaaaa gaaaaaccac cctggcgccc aatacgcaaa 2280

ccgcctctcc ccgcgcgttg gccgattcat taatgcagct ggcacgacag gtttcccgac 2340

tggaaagcgg gcagtgagcg caacgcaatt aatgtaagtt agctcactca ttaggcaccg 2400

ggatctcgac cgatgccctt gagagccttc aacccagtca gctccttccg gtgggcgcgg 2460

ggcatgacta tcgtcgccgc acttatgact gtcttcttta tcatgcaact cgtaggacag 2520

gtgccggcag cgctctgggt cattttcggc gaggaccgct ttcgctggag cgcgacgatg 2580

atcggcctgt cgcttgcggt attcggaatc ttgcacgccc tcgctcaagc cttcgtcact 2640

ggtcccgcca ccaaacgttt cggcgagaag caggccatta tcgccggcat ggcggcccca 2700

cgggtgcgca tgatcgtgct cctgtcgttg aggacccggc taggctggcg gggttgcctt 2760

actggttagc agaatgaatc accgatacgc gagcgaacgt gaagcgactg ctgctgcaaa 2820

acgtctgcga cctgagcaac aacatgaatg gtcttcggtt tccgtgtttc gtaaagtctg 2880

gaaacgcgga agtcagcgcc ctgcaccatt atgttccgga tctgcatcgc aggatgctgc 2940

tggctaccct gtggaacacc tacatctgta ttaacgaagc gctggcattg accctgagtg 3000

atttttctct ggtcccgccg catccatacc gccagttgtt taccctcaca acgttccagt 3060

aaccgggcat gttcatcatc agtaacccgt atcgtgagca tcctctctcg tttcatcggt 3120

atcattaccc ccatgaacag aaatccccct tacacggagg catcagtgac caaacaggaa 3180

aaaaccgccc ttaacatggc ccgctttatc agaagccaga cattaacgct tctggagaaa 3240

ctcaacgagc tggacgcgga tgaacaggca gacatctgtg aatcgcttca cgaccacgct 3300

gatgagcttt accgcagctg cctcgcgcgt ttcggtgatg acggtgaaaa cctctgacac 3360

atgcagctcc cggagacggt cacagcttgt ctgtaagcgg atgccgggag cagacaagcc 3420

cgtcagggcg cgtcagcggg tgttggcggg tgtcggggcg cagccatgac ccagtcacgt 3480

agcgatagcg gagtgtatac tggcttaact atgcggcatc agagcagatt gtactgagag 3540

tgcaccatat atgcggtgtg aaataccgca cagatgcgta aggagaaaat accgcatcag 3600

gcgctcttcc gcttcctcgc tcactgactc gctgcgctcg gtcgttcggc tgcggcgagc 3660

ggtatcagct cactcaaagg cggtaatacg gttatccaca gaatcagggg ataacgcagg 3720

aaagaacatg tgagcaaaag gccagcaaaa ggccaggaac cgtaaaaagg ccgcgttgct 3780

ggcgtttttc cataggctcc gcccccctga cgagcatcac aaaaatcgac gctcaagtca 3840

gaggtggcga aacccgacag gactataaag ataccaggcg tttccccctg gaagctccct 3900

cgtgcgctct cctgttccga ccctgccgct taccggatac ctgtccgcct ttctcccttc 3960

gggaagcgtg gcgctttctc atagctcacg ctgtaggtat ctcagttcgg tgtaggtcgt 4020

tcgctccaag ctgggctgtg tgcacgaacc ccccgttcag cccgaccgct gcgccttatc 4080

cggtaactat cgtcttgagt ccaacccggt aagacacgac ttatcgccac tggcagcagc 4140

cactggtaac aggattagca gagcgaggta tgtaggcggt gctacagagt tcttgaagtg 4200

gtggcctaac tacggctaca ctagaaggac agtatttggt atctgcgctc tgctgaagcc 4260

agttaccttc ggaaaaagag ttggtagctc ttgatccggc aaacaaacca ccgctggtag 4320

cggtggtttt tttgtttgca agcagcagat tacgcgcaga aaaaaaggat ctcaagaaga 4380

tcctttgatc ttttctacgg ggtctgacgc tcagtggaac gaaaactcac gttaagggat 4440

tttggtcatg aacaataaaa ctgtctgctt acataaacag taatacaagg ggtgttatga 4500

gccatattca acgggaaacg tcttgctcta ggccgcgatt aaattccaac atggatgctg 4560

atttatatgg gtataaatgg gctcgcgata atgtcgggca atcaggtgcg acaatctatc 4620

gattgtatgg gaagcccgat gcgccagagt tgtttctgaa acatggcaaa ggtagcgttg 4680

ccaatgatgt tacagatgag atggtcagac taaactggct gacggaattt atgcctcttc 4740

cgaccatcaa gcattttatc cgtactcctg atgatgcatg gttactcacc actgcgatcc 4800

ccgggaaaac agcattccag gtattagaag aatatcctga ttcaggtgaa aatattgttg 4860

atgcgctggc agtgttcctg cgccggttgc attcgattcc tgtttgtaat tgtcctttta 4920

acagcgatcg cgtatttcgt ctcgctcagg cgcaatcacg aatgaataac ggtttggttg 4980

atgcgagtga ttttgatgac gagcgtaatg gctggcctgt tgaacaagtc tggaaagaaa 5040

tgcataaact tttgccattc tcaccggatt cagtcgtcac tcatggtgat ttctcacttg 5100

ataaccttat ttttgacgag gggaaattaa taggttgtat tgatgttgga cgagtcggaa 5160

tcgcagaccg ataccaggat cttgccatcc tatggaactg cctcggtgag ttttctcctt 5220

cattacagaa acggcttttt caaaaatatg gtattgataa tcctgatatg aataaattgc 5280

agtttcattt gatgctcgat gagtttttct aagaattaat tcatgagcgg atacatattt 5340

gaatgtattt agaaaaataa acaaataggg gttccgcgca catttccccg aaaagtgcca 5400

cctgaaattg taaacgttaa tattttgtta aaattcgcgt taaatttttg ttaaatcagc 5460

tcatttttta accaataggc cgaaatcggc aaaatccctt ataaatcaaa agaatagacc 5520

gagatagggt tgagtgttgt tccagtttgg aacaagagtc cactattaaa gaacgtggac 5580

tccaacgtca aagggcgaaa aaccgtctat cagggcgatg gcccactacg tgaaccatca 5640

ccctaatcaa gttttttggg gtcgaggtgc cgtaaagcac taaatcggaa ccctaaaggg 5700

agcccccgat ttagagcttg acggggaaag ccggcgaacg tggcgagaaa ggaagggaag 5760

aaagcgaaag gagcgggcgc tagggcgctg gcaagtgtag cggtcacgct gcgcgtaacc 5820

accacacccg ccgcgcttaa tgcgccgcta cagggcgcgt cccattcgcc a 5871

<210> 4
<211> 3110
<212> DNA
<213> Artificial Sequence

<220>
<223> Vector pUC57ORPHI.

<400> 4
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cgagctcggt acctcgcgaa 420

tgcatctaga tctcgtgcct cctatgattg gttgtcttat taccttactt ctattatagt 480

ataacatgtt aaacgatagt ttgtctaccc ttttcgacaa attgatgata ataaatagta 540

taggtatata gtcgtgattt agttgttaga ttcttgtcga agatagtcgg tcaatgggga 600

aatggtgtat gttgtcgctg taccctactt taaagtaagc ccccaccctc acatgatacc 660

attctcctaa tatcgacata atccgtcgat cctcggcata ccatgatcag ggagggaaac 720

tactacttaa tatatcaatc tatagaccta ctagataggt ttgtcaatga acaacataaa 780

acgacacaga atcccacgtt ttagcgcttc gtctgtgtcg cacagccact gatcggatcc 840

cgggcccgtc gactgcagag gcctgcatgc aagcttggcg taatcatggt catagctgtt 900

tcctgtgtga aattgttatc cgctcacaat tccacacaac atacgagccg gaagcataaa 960

gtgtaaagcc tggggtgcct aatgagtgag ctaactcaca ttaattgcgt tgcgctcact 1020

gcccgctttc cagtcgggaa acctgtcgtg ccagctgcat taatgaatcg gccaacgcgc 1080

ggggagaggc ggtttgcgta ttgggcgctc ttccgcttcc tcgctcactg actcgctgcg 1140

ctcggtcgtt cggctgcggc gagcggtatc agctcactca aaggcggtaa tacggttatc 1200

cacagaatca ggggataacg caggaaagaa catgtgagca aaaggccagc aaaaggccag 1260

gaaccgtaaa aaggccgcgt tgctggcgtt tttccatagg ctccgccccc ctgacgagca 1320

tcacaaaaat cgacgctcaa gtcagaggtg gcgaaacccg acaggactat aaagatacca 1380

ggcgtttccc cctggaagct ccctcgtgcg ctctcctgtt ccgaccctgc cgcttaccgg 1440

atacctgtcc gcctttctcc cttcgggaag cgtggcgctt tctcatagct cacgctgtag 1500

gtatctcagt tcggtgtagg tcgttcgctc caagctgggc tgtgtgcacg aaccccccgt 1560

tcagcccgac cgctgcgcct tatccggtaa ctatcgtctt gagtccaacc cggtaagaca 1620

cgacttatcg ccactggcag cagccactgg taacaggatt agcagagcga ggtatgtagg 1680

cggtgctaca gagttcttga agtggtggcc taactacggc tacactagaa gaacagtatt 1740

tggtatctgc gctctgctga agccagttac cttcggaaaa agagttggta gctcttgatc 1800

cggcaaacaa accaccgctg gtagcggtgg tttttttgtt tgcaagcagc agattacgcg 1860

cagaaaaaaa ggatctcaag aagatccttt gatcttttct acggggtctg acgctcagtg 1920

gaacgaaaac tcacgttaag ggattttggt catgagatta tcaaaaagga tcttcaccta 1980

gatcctttta aattaaaaat gaagttttaa atcaatctaa agtatatatg agtaaacttg 2040

gtctgacagt taccaatgct taatcagtga ggcacctatc tcagcgatct gtctatttcg 2100

ttcatccata gttgcctgac tccccgtcgt gtagataact acgatacggg agggcttacc 2160

atctggcccc agtgctgcaa tgataccgcg agacccacgc tcaccggctc cagatttatc 2220

agcaataaac cagccagccg gaagggccga gcgcagaagt ggtcctgcaa ctttatccgc 2280

ctccatccag tctattaatt gttgccggga agctagagta agtagttcgc cagttaatag 2340

tttgcgcaac gttgttgcca ttgctacagg catcgtggtg tcacgctcgt cgtttggtat 2400

ggcttcattc agctccggtt cccaacgatc aaggcgagtt acatgatccc ccatgttgtg 2460

caaaaaagcg gttagctcct tcggtcctcc gatcgttgtc agaagtaagt tggccgcagt 2520

gttatcactc atggttatgg cagcactgca taattctctt actgtcatgc catccgtaag 2580

atgcttttct gtgactggtg agtactcaac caagtcattc tgagaatagt gtatgcggcg 2640

accgagttgc tcttgcccgg cgtcaatacg ggataatacc gcgccacata gcagaacttt 2700

aaaagtgctc atcattggaa aacgttcttc ggggcgaaaa ctctcaagga tcttaccgct 2760

gttgagatcc agttcgatgt aacccactcg tgcacccaac tgatcttcag catcttttac 2820

tttcaccagc gtttctgggt gagcaaaaac aggaaggcaa aatgccgcaa aaaagggaat 2880

aagggcgaca cggaaatgtt gaatactcat actcttcctt tttcaatatt attgaagcat 2940

ttatcagggt tattgtctca tgagcggata catatttgaa tgtatttaga aaaataaaca 3000

aataggggtt ccgcgcacat ttccccgaaa agtgccacct gacgtctaag aaaccattat 3060

tatcatgaca ttaacctata aaaataggcg tatcacgagg ccctttcgtc 3110

<210> 5
<211> 68
<212> DNA
<213> Bacteriophage phi-29

<220>
<221> variation
<222> (1)..(1)
<223> La adenina en la posicion 1 esta fosforilada en 5'.

<220>
<221>  variation
<222>  (1)..(1)
<223>  La adenina en la posicion 1 lleva un grupo propilo.

<220>
<221>  variation
<222>  (1)..(1)
<223>  La adenina en la posicion 1 esta biotinilada.

<400>  5
aaagtaagcc cccaccctca catgatacca ttctcctaat atcgacataa tccgtcgatc      60

ctcggcat                                                              68


<210>  6
<211>  72
<212>  DNA
<213>  Artificial Sequence

<220>
<223>  Oligonucleotido con los 68 nucleotidos del extremo izquierdo del ADN
del bacteriofago Phi-29 en 3' y el extremo cohesivo de la    diana de EcoRI
en 5'.


<220>
<221>  variation
<222>  (1)..(1)
<223>  La adenina en la posicion 1 esta fosforilada en 5'.

<400>  6
aattatgccg aggatcgacg gattatgtcg atattaggag aatggtatca tgtgagggtg      60

ggggcttact tt                                                          72


<210>  7
<211>  72
<212>  DNA
<213>  Artificial Sequence

<220>
<223>  Oligonucleotido con los 68 nucleotidos del extremo izquierdo del ADN
del bacteriofago Phi-29 en 3' y el extremo cohesivo de la        diana de
BsmBI en 5'.


<220>
<221>  variation
<222>  (1)..(1)
<223>  La adenina en la posicion 1 esta fosforilada en 5'.

<400>  7
atccatgccg aggatcgacg gattatgtcg atattaggag aatggtatca tgtgagggtg      60

ggggcttact tt                                                          72


<210>  8
<211>  71
<212>  DNA

<213>  Artificial Sequence

<220>
<223>  Oligonucleotido con los 68 nucleotidos del extremo izquierdo del ADN
del bacteriofago Phi-29 en 3' y el extremo cohesivo de la       diana de
Eco109I en 5'.

<220>
<221>  variation
<222>  (1)..(1)
<223>  La guanina en la posicion 1 esta fosforilada en 5'.

<400>  8
gccatgccga ggatcgacgg attatgtcga tattaggaga atggtatcat gtgagggtgg       60

gggcttactt t                                                            71

<210>  9
<211>  38
<212>  DNA
<213>  Artificial Sequence

<220>
<223>  Oligonucleotido directo para generar la secuencia diana de
       restriccion de BaeI insertada en la secuencia diana de
       restriccion de DraI de pETORPHI.

<400>  9
tctcctaatt atttgactgt cgtatctgtc atcaacca                               38

<210>  10
<211>  38
<212>  DNA
<213>  Artificial Sequence

<220>
<223>  Oligonucleotido inverso para generar la secuencia diana de
       restriccion de BaeI insertada en la secuencia diana de
       restriccion de DraI en pETORPHI.

<400>  10
tggttgatga cagatacgac agtcaaataa ttaggaga                               38

<210>  11
<211>  5368
<212>  DNA
<213>  Artificial Sequence

<220>
<223>  Vector pET28b.

<400>  11
atccggatat agttcctcct ttcagcaaaa aacccctcaa gacccgttta gaggccccaa       60

ggggttatgc tagttattgc tcagcggtgg cagcagccaa ctcagcttcc tttcgggctt      120

tgttagcagc cggatctcag tggtggtggt ggtggtgctc gagtgcggcc gcaagcttgt      180

cgacggagct cgaattcgga tcccgaccca tttgctgtcc accagtcatg ctagccatat      240

ggctgccgcg cggcaccagg ccgctgctgt gatgatgatg atgatggctg ctgcccatgg 300

tatatctcct tcttaaagtt aaacaaaatt atttctagag gggaattgtt atccgctcac 360

aattccccta tagtgagtcg tattaatttc gcgggatcga gatctcgatc ctctacgccg 420

gacgcatcgt ggccggcatc accggcgcca caggtgcggt tgctggcgcc tatatcgccg 480

acatcaccga tggggaagat cgggctcgcc acttcgggct catgagcgct tgtttcggcg 540

tgggtatggt ggcaggcccc gtggccgggg gactgttggg cgccatctcc ttgcatgcac 600

cattccttgc ggcggcggtg ctcaacggcc tcaacctact actgggctgc ttcctaatgc 660

aggagtcgca taagggagag cgtcgagatc ccggacacca tcgaatggcg caaaaccttt 720

cgcggtatgg catgatagcg cccggaagag agtcaattca gggtggtgaa tgtgaaacca 780

gtaacgttat acgatgtcgc agagtatgcc ggtgtctctt atcagaccgt ttcccgcgtg 840

gtgaaccagg ccagccacgt ttctgcgaaa acgcgggaaa aagtggaagc ggcgatggcg 900

gagctgaatt acattcccaa ccgcgtggca caacaactgg cgggcaaaca gtcgttgctg 960

attggcgttg ccacctccag tctggccctg cacgcgccgt cgcaaattgt cgcggcgatt 1020

aaatctcgcg ccgatcaact gggtgccagc gtggtggtgt cgatggtaga acgaagcggc 1080

gtcgaagcct gtaaagcggc ggtgcacaat cttctcgcgc aacgcgtcag tgggctgatc 1140

attaactatc cgctggatga ccaggatgcc attgctgtgg aagctgcctg cactaatgtt 1200

ccggcgttat ttcttgatgt ctctgaccag acacccatca acagtattat tttctcccat 1260

gaagacggta cgcgactggg cgtggagcat ctggtcgcat tgggtcacca gcaaatcgcg 1320

ctgttagcgg gcccattaag ttctgtctcg gcgcgtctgc gtctggctgg ctggcataaa 1380

tatctcactc gcaatcaaat tcagccgata gcggaacggg aaggcgactg gagtgccatg 1440

tccggttttc aacaaaccat gcaaatgctg aatgagggca tcgttcccac tgcgatgctg 1500

gttgccaacg atcagatggc gctgggcgca atgcgcgcca ttaccgagtc cgggctgcgc 1560

gttggtgcgg atatctcggt agtgggatac gacgataccg aagacagctc atgttatatc 1620

ccgccgttaa ccaccatcaa acaggatttt cgcctgctgg ggcaaaccag cgtggaccgc 1680

ttgctgcaac tctctcaggg ccaggcggtg aagggcaatc agctgttgcc cgtctcactg 1740

gtgaaaagaa aaaccaccct ggcgcccaat acgcaaaccg cctctccccg cgcgttggcc 1800

gattcattaa tgcagctggc acgacaggtt tcccgactgg aaagcgggca gtgagcgcaa 1860

cgcaattaat gtaagttagc tcactcatta ggcaccggga tctcgaccga tgcccttgag 1920

agccttcaac ccagtcagct ccttccggtg ggcgcggggc atgactatcg tcgccgcact 1980

tatgactgtc ttctttatca tgcaactcgt aggacaggtg ccggcagcgc tctgggtcat 2040

tttcggcgag gaccgctttc gctggagcgc gacgatgatc ggcctgtcgc ttgcggtatt 2100

cggaatcttg cacgccctcg ctcaagcctt cgtcactggt cccgccacca aacgtttcgg 2160

cgagaagcag gccattatcg ccggcatggc ggccccacgg gtgcgcatga tcgtgctcct 2220

gtcgttgagg acccggctag gctggcgggg ttgccttact ggttagcaga atgaatcacc 2280

gatacgcgag cgaacgtgaa gcgactgctg ctgcaaaacg tctgcgacct gagcaacaac 2340

atgaatggtc ttcggtttcc gtgtttcgta aagtctggaa acgcggaagt cagcgccctg 2400

caccattatg ttccggatct gcatcgcagg atgctgctgg ctaccctgtg gaacacctac 2460

atctgtatta acgaagcgct ggcattgacc ctgagtgatt tttctctggt cccgccgcat 2520

ccataccgcc agttgtttac cctcacaacg ttccagtaac cgggcatgtt catcatcagt 2580

aacccgtatc gtgagcatcc tctctcgttt catcggtatc attaccccca tgaacagaaa 2640

tcccccttac acggaggcat cagtgaccaa acaggaaaaa accgccctta acatggcccg 2700

ctttatcaga agccagacat taacgcttct ggagaaactc aacgagctgg acgcggatga 2760

acaggcagac atctgtgaat cgcttcacga ccacgctgat gagctttacc gcagctgcct 2820

cgcgcgtttc ggtgatgacg gtgaaaacct ctgacacatg cagctcccgg agacggtcac 2880

agcttgtctg taagcggatg ccgggagcag acaagcccgt cagggcgcgt cagcgggtgt 2940

tggcgggtgt cggggcgcag ccatgaccca gtcacgtagc gatagcggag tgtatactgg 3000

cttaactatg cggcatcaga gcagattgta ctgagagtgc accatatatg cggtgtgaaa 3060

taccgcacag atgcgtaagg agaaaatacc gcatcaggcg ctcttccgct tcctcgctca 3120

ctgactcgct gcgctcggtc gttcggctgc ggcgagcggt atcagctcac tcaaaggcgg 3180

taatacggtt atccacagaa tcaggggata acgcaggaaa gaacatgtga gcaaaaggcc 3240

agcaaaaggc caggaaccgt aaaaaggccg cgttgctggc gtttttccat aggctccgcc 3300

cccctgacga gcatcacaaa aatcgacgct caagtcagag gtggcgaaac ccgacaggac 3360

tataaagata ccaggcgttt ccccctggaa gctccctcgt gcgctctcct gttccgaccc 3420

tgccgcttac cggatacctg tccgcctttc tcccttcggg aagcgtggcg ctttctcata 3480

gctcacgctg taggtatctc agttcggtgt aggtcgttcg ctccaagctg ggctgtgtgc 3540

acgaaccccc cgttcagccc gaccgctgcg ccttatccgg taactatcgt cttgagtcca 3600

acccggtaag acacgactta tcgccactgg cagcagccac tggtaacagg attagcagag 3660

cgaggtatgt aggcggtgct acagagttct tgaagtggtg gcctaactac ggctacacta 3720

gaaggacagt atttggtatc tgcgctctgc tgaagccagt taccttcgga aaaagagttg 3780

gtagctcttg atccggcaaa caaaccaccg ctggtagcgg tggttttttt gtttgcaagc 3840

agcagattac gcgcagaaaa aaaggatctc aagaagatcc tttgatcttt tctacggggt 3900

ctgacgctca gtggaacgaa aactcacgtt aagggatttt ggtcatgaac aataaaactg 3960

tctgcttaca taaacagtaa tacaaggggt gttatgagcc atattcaacg ggaaacgtct 4020

tgctctaggc cgcgattaaa ttccaacatg gatgctgatt tatatgggta taaatgggct 4080

cgcgataatg tcgggcaatc aggtgcgaca atctatcgat tgtatgggaa gcccgatgcg 4140

ccagagttgt ttctgaaaca tggcaaaggt agcgttgcca atgatgttac agatgagatg 4200

gtcagactaa actggctgac ggaatttatg cctcttccga ccatcaagca ttttatccgt 4260

actcctgatg atgcatggtt actcaccact gcgatccccg ggaaaacagc attccaggta 4320

ttagaagaat atcctgattc aggtgaaaat attgttgatg cgctggcagt gttcctgcgc 4380

cggttgcatt cgattcctgt ttgtaattgt ccttttaaca gcgatcgcgt atttcgtctc 4440

gctcaggcgc aatcacgaat gaataacggt ttggttgatg cgagtgattt tgatgacgag 4500

cgtaatggct ggcctgttga acaagtctgg aaagaaatgc ataaactttt gccattctca 4560

ccggattcag tcgtcactca tggtgatttc tcacttgata accttatttt tgacgagggg 4620

aaattaatag gttgtattga tgttggacga gtcggaatcg cagaccgata ccaggatctt 4680

gccatcctat ggaactgcct cggtgagttt tctccttcat tacagaaacg gctttttcaa 4740

aaatatggta ttgataatcc tgatatgaat aaattgcagt ttcatttgat gctcgatgag 4800

tttttctaag aattaattca tgagcggata catatttgaa tgtatttaga aaaataaaca 4860

aataggggtt ccgcgcacat ttccccgaaa agtgccacct gaaattgtaa acgttaatat 4920

tttgttaaaa ttcgcgttaa atttttgtta aatcagctca ttttttaacc aataggccga 4980

aatcggcaaa atcccttata aatcaaaaga atagaccgag atagggttga gtgttgttcc 5040

agtttggaac aagagtccac tattaaagaa cgtggactcc aacgtcaaag ggcgaaaaac 5100

cgtctatcag ggcgatggcc cactacgtga accatcaccc taatcaagtt ttttggggtc 5160

gaggtgccgt aaagcactaa atcggaaccc taaagggagc ccccgattta gagcttgacg 5220

gggaaagccg gcgaacgtgg cgagaaagga agggaagaaa gcgaaaggag cgggcgctag 5280

ggcgctggca agtgtagcgg tcacgctgcg cgtaaccacc acacccgccg cgcttaatgc 5340

gccgctacag ggcgcgtccc attcgcca 5368

<210> 12
<211> 191
<212> DNA
<213> Bacteriophage phi-29

<400> 12
aaagtaagcc cccaccctca catgatacca ttctcctaat atcgacataa tccgtcgatc 60

ctcggcatac catgatcagg gagggaaact actacttaat atatcaatct atagacctac 120

tagataggtt tgtcaatgaa caacataaaa cgacacagaa tcccacgttt tagcgcttcg 180

tctgtgtcgc a 191

<210> 13
<211> 194
<212> DNA
<213> Bacteriophage phi-29

<400> 13
aaagtagggt acagcgacaa catacaccat ttccccattg accgactatc ttcgacaaga 60

atctaacaac taaatcacga ctatatacct atactattta ttatcatcaa tttgtcgaaa 120

agggtagaca aactatcgtt taacatgtta tactataata gaagtaaggt aataagacaa 180

ccaatcatag gagg 194

<210> 14
<211> 4733
<212> DNA
<213> Artificial Sequence

<220>
<223> pEYFP-N1

<400> 14
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660

gatccaccgg tcgccaccat ggtgagcaag ggcgaggagc tgttcaccgg ggtggtgccc 720

atcctggtcg agctggacgg cgacgtaaac ggccacaagt tcagcgtgtc cggcgagggc 780

gagggcgatg ccacctacgg caagctgacc ctgaagttca tctgcaccac cggcaagctg 840

cccgtgccct ggcccaccct cgtgaccacc ttcggctacg gcctgcagtg cttcgcccgc 900

taccccgacc acatgaagca gcacgacttc ttcaagtccg ccatgcccga aggctacgtc 960

caggagcgca ccatcttctt caaggacgac ggcaactaca agacccgcgc cgaggtgaag 1020

ttcgagggcg acaccctggt gaaccgcatc gagctgaagg gcatcgactt caaggaggac 1080

ggcaacatcc tggggcacaa gctggagtac aactacaaca gccacaacgt ctatatcatg 1140

gccgacaagc agaagaacgg catcaaggtg aacttcaaga tccgccacaa catcgaggac 1200

ggcagcgtgc agctcgccga ccactaccag cagaacaccc ccatcggcga cggccccgtg 1260

ctgctgcccg acaaccacta cctgagctac cagtccgccc tgagcaaaga ccccaacgag 1320

aagcgcgatc acatggtcct gctggagttc gtgaccgccg ccgggatcac tctcggcatg 1380

gacgagctgt acaagtaaag cggccgcgac tctagatcat aatcagccat accacatttg 1440

tagaggtttt acttgcttta aaaaacctcc cacacctccc cctgaacctg aaacataaaa 1500

tgaatgcaat tgttgttgtt aacttgttta ttgcagctta taatggttac aaataaagca 1560

atagcatcac aaatttcaca aataaagcat ttttttcact gcattctagt tgtggtttgt 1620

ccaaactcat caatgtatct taaggcgtaa attgtaagcg ttaatatttt gttaaaattc 1680

gcgttaaatt tttgttaaat cagctcattt tttaaccaat aggccgaaat cggcaaaatc 1740

ccttataaat caaaagaata gaccgagata gggttgagtg ttgttccagt ttggaacaag 1800

agtccactat taaagaacgt ggactccaac gtcaaagggc gaaaaaccgt ctatcagggc 1860

gatggcccac tacgtgaacc atcaccctaa tcaagttttt tggggtcgag gtgccgtaaa 1920

gcactaaatc ggaaccctaa agggagcccc cgatttagag cttgacgggg aaagccggcg 1980

aacgtggcga gaaaggaagg gaagaaagcg aaaggagcgg gcgctagggc gctggcaagt 2040

gtagcggtca cgctgcgcgt aaccaccaca cccgccgcgc ttaatgcgcc gctacagggc 2100

gcgtcaggtg gcacttttcg gggaaatgtg cgcggaaccc ctatttgttt atttttctaa 2160

atacattcaa atatgtatcc gctcatgaga caataaccct gataaatgct tcaataatat 2220

tgaaaaagga agagtcctga ggcggaaaga accagctgtg gaatgtgtgt cagttagggt 2280

gtggaaagtc cccaggctcc ccagcaggca gaagtatgca aagcatgcat ctcaattagt 2340

cagcaaccag gtgtggaaag tccccaggct ccccagcagg cagaagtatg caaagcatgc 2400

atctcaatta gtcagcaacc atagtcccgc ccctaactcc gcccatcccg cccctaactc 2460

cgcccagttc cgcccattct ccgccccatg gctgactaat tttttttatt tatgcagagg 2520

ccgaggccgc ctcggcctct gagctattcc agaagtagtg aggaggcttt tttggaggcc 2580

taggcttttg caaagatcga tcaagagaca ggatgaggat cgtttcgcat gattgaacaa 2640

gatggattgc acgcaggttc tccggccgct tgggtggaga ggctattcgg ctatgactgg 2700

gcacaacaga caatcggctg ctctgatgcc gccgtgttcc ggctgtcagc gcaggggcgc 2760

ccggttcttt ttgtcaagac cgacctgtcc ggtgccctga atgaactgca agacgaggca 2820

gcgcggctat cgtggctggc cacgacgggc gttccttgcg cagctgtgct cgacgttgtc 2880

actgaagcgg gaagggactg gctgctattg ggcgaagtgc cggggcagga tctcctgtca 2940

tctcaccttg ctcctgccga gaaagtatcc atcatggctg atgcaatgcg gcggctgcat 3000

acgcttgatc cggctacctg cccattcgac caccaagcga aacatcgcat cgagcgagca 3060

cgtactcgga tggaagccgg tcttgtcgat caggatgatc tggacgaaga gcatcagggg 3120

ctcgcgccag ccgaactgtt cgccaggctc aaggcgagca tgcccgacgg cgaggatctc 3180

gtcgtgaccc atggcgatgc ctgcttgccg aatatcatgg tggaaaatgg ccgcttttct 3240

ggattcatcg actgtggccg gctgggtgtg gcggaccgct atcaggacat agcgttggct 3300

acccgtgata ttgctgaaga gcttggcggc gaatgggctg accgcttcct cgtgctttac 3360

ggtatcgccg ctcccgattc gcagcgcatc gccttctatc gccttcttga cgagttcttc 3420

tgagcgggac tctggggttc gaaatgaccg accaagcgac gcccaacctg ccatcacgag 3480

atttcgattc caccgccgcc ttctatgaaa ggttgggctt cggaatcgtt ttccgggacg 3540

ccggctggat gatcctccag cgcggggatc tcatgctgga gttcttcgcc caccctaggg 3600

ggaggctaac tgaaacacgg aaggagacaa taccggaagg aacccgcgct atgacggcaa 3660

taaaaagaca gaataaaacg cacggtgttg ggtcgtttgt tcataaacgc ggggttcggt 3720

cccagggctg gcactctgtc gataccccac cgagacccca ttggggccaa tacgcccgcg 3780

tttcttcctt ttccccaccc caccccccaa gttcgggtga aggcccaggg ctcgcagcca 3840

acgtcggggc ggcaggccct gccatagcct caggttactc atatatactt tagattgatt 3900

taaaacttca tttttaattt aaaaggatct aggtgaagat cctttttgat aatctcatga 3960

ccaaaatccc ttaacgtgag ttttcgttcc actgagcgtc agaccccgta gaaaagatca 4020

aaggatcttc ttgagatcct ttttttctgc gcgtaatctg ctgcttgcaa acaaaaaaac 4080

caccgctacc agcggtggtt tgtttgccgg atcaagagct accaactctt tttccgaagg 4140

taactggctt cagcagagcg cagataccaa atactgtcct tctagtgtag ccgtagttag 4200

gccaccactt caagaactct gtagcaccgc ctacatacct cgctctgcta atcctgttac 4260

cagtggctgc tgccagtggc gataagtcgt gtcttaccgg gttggactca agacgatagt 4320

taccggataa ggcgcagcgg tcgggctgaa cggggggttc gtgcacacag cccagcttgg 4380

agcgaacgac ctacaccgaa ctgagatacc tacagcgtga gctatgagaa agcgccacgc 4440

ttcccgaagg gagaaaggcg gacaggtatc cggtaagcgg cagggtcgga acaggagagc 4500

gcacgaggga gcttccaggg ggaaacgcct ggtatcttta tagtcctgtc gggtttcgcc 4560

acctctgact tgagcgtcga tttttgtgat gctcgtcagg ggggcggagc ctatggaaaa 4620

acgccagcaa cgcggccttt ttacggttcc tggccttttg ctggcctttt gctcacatgt 4680

tctttcctgc gttatcccct gattctgtgg ataaccgtat taccgccatg cat 4733

<210> 15
<211> 4733
<212> DNA
<213> Artificial Sequence

<220>

<223>  pEYFP-N1Bsm

<400>  15
tagttattaa tagtaatcaa ttacggggtc attagttcat agcccatata tggagttccg      60

cgttacataa cttacggtaa atggcccgcc tggctgaccg cccaacgacc cccgcccatt     120

gacgtcaata atgacgtatg ttcccatagt aacgccaata gggactttcc attgacgtca     180

atgggtggag tatttacggt aaactgccca cttggcagta catcaagtgt atcatatgcc     240

aagtacgccc cctattgacg tcaatgacgg taaatggccc gcctggcatt atgcccagta     300

catgacctta tgggactttc ctacttggca gtacatctac gtattagtca tcgctattac     360

catggtgatg cggttttggc agtacatcaa tgggcgtgga tagcggtttg actcacgggg     420

atttccaagt ctccacccca ttgacgtcaa tgggagtttg ttttggcacc aaaatcaacg     480

ggactttcca aaatgtcgta acaactccgc cccattgacg caaatgggcg gtaggcgtgt     540

acggtgggag gtctatataa gcagagctgg tttagtgaac cgtcagatcc gctagcgcta     600

ccggactcag atctcgagct caagcttcga attctgcagt cgacggtacc gcgggcccgg     660

gatccaccgg tcgccaccat ggtgagcaag ggcgaggagc tgttcaccgg ggtggtgccc     720

atcctggtcg agctggacgg cgacgtaaac ggccacaagt tcagcgtgtc cggcgagggc     780

gagggcgatg ccacctacgg caagctgacc ctgaagttca tctgcaccac cggcaagctg     840

cccgtgccct ggcccaccct cgtgaccacc ttcggctacg gcctgcagtg cttcgcccgc     900

taccccgacc acatgaagca gcacgacttc ttcaagtccg ccatgcccga aggctacgtc     960

caggagcgca ccatcttctt caaggacgac ggcaactaca agacccgcgc cgaggtgaag    1020

ttcgagggcg acaccctggt gaaccgcatc gagctgaagg gcatcgactt caaggaggac    1080

ggcaacatcc tggggcacaa gctggagtac aactacaaca gccacaacgt ctatatcatg    1140

gccgacaagc agaagaacgg catcaaggtg aacttcaaga tccgccacaa catcgaggac    1200

ggcagcgtgc agctcgccga ccactaccag cagaacaccc ccatcggcga cggccccgtg    1260

ctgctgcccg acaaccacta cctgagctac cagtccgccc tgagcaaaga ccccaacgag    1320

aagcgcgatc acatggtcct gctggagttc gtgaccgccg ccgggatcac tctcggcatg    1380

gacgagctgt acaagtaaag cggccgcgac tctagatcat aatcagccat accacatttg    1440

tagaggtttt acttgcttta aaaaacctcc cacacctccc cctgaacctg aaacataaaa    1500

tgaatgcaat tgttgttgtt aacttgttta ttgcagctta taatggttac aaataaagca    1560

atagcatcac aaatttcaca aataaagcat ttttttcact gcattctagt tgtggtttgt    1620

ccaaactcat caatgtatct taaggcgtaa attgtaagcg ttaatatttt gttaaaattc    1680

gcgttaaatt tttgttaaat cagctcattt tttaaccaat aggccgaaat cggcaaaatc    1740

ccttataaat caaaagaata gaccgagata gggttgagtg ttgttccagt ttggaacaag    1800

agtccactat taaagaacgt ggactccaac gtcaaagggc gaaaaaccgt ctatcagggc 1860

gatggcccac tacgtgaacc atcaccctaa tcaagttttt tggggtcgag gtgccgtaaa 1920

gcactaaatc ggaaccctaa agggagcccc cgatttagag cttgacgggg aaagccggcg 1980

aacgtggcga gaaaggaagg gaagaaagcg aaaggagcgg gcgctagggc gctggcaagt 2040

gtagcggtca cgctgcgcgt aaccaccaca cccgccgcgc ttaatgcgcc gctacagggc 2100

gcgtcaggtg gcacttttcg gggaaatgtg cgcggaaccc ctatttgttt atttttctaa 2160

atacattcaa atatgtatcc gctcatgaga caataaccct gataaatgct tcaataatat 2220

tgaaaaagga agagtcctga ggcggaaaga accagctgtg gaatgtgtgt cagttagggt 2280

gtggaaagtc cccaggctcc ccagcaggca gaagtatgca aagcatgcat ctcaattagt 2340

cagcaaccag gtgtggaaag tccccaggct ccccagcagg cagaagtatg caaagcatgc 2400

atctcaatta gtcagcaacc atagtcccgc ccctaactcc gcccatcccg cccctaactc 2460

cgcccagttc cgcccattct ccgccccatg gctgactaat tttttttatt tatgcagagg 2520

ccgaggccgc ctcggcctct gagctattcc agaagtagtg aggaggcttt tttggaggcc 2580

taggcttttg caaagatcga tcaagagaca ggatgaggat cgtttcgcat gattgaacaa 2640

gatggattgc acgcaggttc tccggccgct tgggtggaga ggctattcgg ctatgactgg 2700

gcacaacaga caatcggctg ctctgatgcc gccgtgttcc ggctgtcagc gcaggggcgc 2760

ccggttcttt ttgtcaagac cgacctgtcc ggtgccctga atgaactgca agacgaggca 2820

gcgcggctat cgtggctggc cacgacgggc gttccttgcg cagctgtgct cgacgttgtc 2880

actgaagcgg gaagggactg gctgctattg ggcgaagtgc cggggcagga tctcctgtca 2940

tctcaccttg ctcctgccga gaaagtatcc atcatggctg atgcaatgcg gcggctgcat 3000

acgcttgatc cggctacctg cccattcgac caccaagcga aacatcgcat cgagcgagca 3060

cgtactcgga tggaagccgg tcttgtcgat caggatgatc tggacgaaga gcatcagggg 3120

ctcgcgccag ccgaactgtt cgccaggctc aaggcgagca tgcccgacgg cgaggatctc 3180

gtcgtgaccc atggcgatgc ctgcttgccg aatatcatgg tggaaaatgg ccgcttttct 3240

ggattcatcg actgtggccg gctgggtgtg gcggaccgct atcaggacat agcgttggct 3300

acccgtgata ttgctgaaga gcttggcggc gaatgggctg accgcttcct cgtgctttac 3360

ggtatcgccg ctcccgattc gcagcgcatc gccttctatc gccttcttga cgagttcttc 3420

tgagcgggac tctggggttc gaaatgaccg accaagcgac gcccaacctg ccatcacgag 3480

atttcgattc caccgccgcc ttctatgaaa ggttgggctt cggaatcgtt ttccgggacg 3540

ccggctggat gatcctccag cgcggggatc tcatgctgga gttcttcgcc caccctaggg 3600

ggaggctaac tgaaacacgg aaggagacaa taccggaagg aacccgcgct atgacggcaa 3660

taaaaagaca gaataaaacg cacggtgttg ggtcgtttgt tcataaacgc ggggttcggt 3720

cccagggctg gcactctgtc gataccccac cgagacccca ttggggccaa tacgcccgcg 3780

tttcttcctt ttccccaccc caccccccaa gttcgggtga aggcccaggg ctcgcagcca 3840

acgtcggggc ggcaggccct gccatagcct caggttactc atatatactt tagattgatt 3900

taaaacttca tttttaattt aaaaggatct aggtgaagat cctttttgat aatctcatga 3960

ccaaaatccc ttaacgtgag ttttcgttcc actgagcgtc agaccccgta gaaaagatca 4020

aaggatcttc ttgagatcct ttttttctgc gcgtaatctg ctgcttgcaa acaaaaaaac 4080

caccgctacc agcggtggtt tgtttgccgg atcaagagct accaactctt tttccgaagg 4140

taactggctt cagcagagcg cagataccaa atactgtcct tctagtgtag ccgtagttag 4200

gccaccactt caagaactct gtagcaccgc ctacatacct cgctctgcta atcctgttac 4260

cagtggctgc tgccagtggc gataagtcgt gtcttaccgg gttggactca agacgatagt 4320

taccggataa ggcgcagcgg tcgggctgaa cggggggttc gtgcacacag cccagcttgg 4380

agcgaacgac ctacaccgaa ctgagatacc tacagcgtga gctatgagaa agcgccacgc 4440

ttcccgaagg gagaaaggcg gacaggtatc cggtaagcgg cagggtcgga acaggagagc 4500

gcacgaggga gcttccaggg ggaaacgcct ggtatcttta tagtcctgtc gggtttcgcc 4560

acctctgact tgagcgtcga tttttgtgat gctcgtcagg ggggcggagc ctatggaaaa 4620

acgccagcaa cgcggccttt ttacggttcc tggccttttg ctggcctttt gctcacatgt 4680

tctttcctgc gttatcccct gcgtctctgg ataaccgtat taccgccatg cat 4733

## Description

The present invention corresponds to the field of molecular biology and refers to a DNA amplification method based on the replication origins of bacteriophage ϕ29, and to the genic constructs, vectors and oligonucleotides that can be used in the method for amplifying an exogenous sequence of interest.

### STATE OF THE PREVIOUS TECHNIQUE

Deoxyribonucleic acid amplification methods are of great importance in molecular biology. The most widely used techniques are polymerase chain reaction (PCR) and multiple displacement amplification (MDA). Although PCR has the advantage of being very effective and generates defined DNA segments, it is not able to amplify sequences of more than 20 kilobases (kb) in length.

While the most common methods for starting DNA replication use a DNA or RNA molecule as primer, DNA synthesis can also be started using a protein as primer. As receptor of the first phosphodiester bond, the systems of this latter kind start DNA synthesis using the OH group of a specific serine, threonine or tyrosine residue present in a given protein, instead of the 3' OH group of a ribose. This protein is generally referred to as the terminal protein (TP), since it is covalently bound to the 5'-extremity of the DNA molecule. Studies have been made of a broad series of DNA replication systems involving initiation with TP, such as certain bacteriophages (ϕ29, Nf, GA-1, PRD1 or Cp-1), linear plasmids from bacteria (pSCL and pSLA2), mitochondrial DNA, DNA from yeasts and plants, bacterial chromosomes *(Streptomyces* spp.), and DNA from mammalian viruses (adenovirus) (Salas, M. 1991. Annu. Rev. Biochem. 60, 39-71).

There have also been descriptions of *in vivo* methods for generating linear DNA chains with TP bound to the 5' extremities for plasmids of *Streptomyces* (Shiffman, D. and Cohen, S.N. 1992. Proc Natl Acad Sci USA 89, 6129-33) and for adenovirus (Crouzet, J. et al., 1997 Proc Natl Acad Sci USA 94, 1414-9). These methods are based on the observation that following transformation and selection in the appropriate host, DNA containing the adequate sequences but lacking bound TP can acquire the TP needed for replication and stable maintenance within the cell. The obtainment of TP-DNA implies execution of the adequate cloning steps for the plasmid to generate the signals needed for replication based on the TP. DNA construction then must undergo a cloning and selection process in an adequate host, and finally the DNA must be extracted from the host, followed by purification for the intended application purposes. All these steps can limit the size and restrict the type of sequences that can be cloned, maintained and obtained in a stable manner. To date, none of these systems have been employed to produce substantial amounts of TP-DNA for different uses in molecular biology.

Regarding the *in vitro* approach, the most effective system is based on DNA replication of bacteriophage ϕ29. Gutiérrez et al. (Nucleic Acids Research 1988. 16 (13); 5895-5914) described the analysis of the minimal origins of *in vitro* initiation and replication in the left and right extremities of the DNA of bacteriophage ϕ29, and found these minimal origins to comprise the 12 terminal nucleotides. Within these 12 nucleotides, the three terminal nucleotides cannot be mutated without reducing the efficiency of initiation and replication, while the sequence in less terminal positions does not have to be so exact. Descriptions have also been made of the *in vitro* initiation and replication of DNA based on the replication origins of ϕ29 in the presence of the DNA polymerase of bacteriophage ϕ29 and the TP of ϕ29, though not referred to the amplification of DNA.

W09010064 describes a method for amplifying a DNA sequence of up to several hundred kb flanked by the replication origins of ϕ29 - specifically, the 12 nucleotides of the left extremity of the DNA of ϕ29 on one side and the 12 nucleotides of the right extremity of the DNA of ϕ29 on the other, in the presence of the DNA polymerase of bacteriophage ϕ29 and the TP of ϕ29. W09010064 describes oligonucleotides comprising the 12 nucleotides of the left extremity of the DNA of ϕ29. However, the method described in W09010064 is based on the results of Gutiérrez et al. (1988), which are not referred to DNA amplification but to DNA replication. The results presented by Blanco et al. (Proc. Natl. Acad. Sci. USA. 1994. Vol. 91; 12198-12202) show that the DNA amplification described in W09010064 is not possible; consequently, the description in this patent regarding DNA amplification is wrong, since DNA amplification cannot be carried out with only the 12 nucleotides of the extremity of the DNA of ϕ29.

Blanco et al. (1994) reported that efficient *in vitro* amplification of the DNA of ϕ29 requires the presence not only of the DNA polymerase of bacteriophage ϕ29 and the TP of ϕ29, but also of large amounts of proteins p5 and p6 of ϕ29.

The fact that TP is covalently bound to the genome of ϕ29 destined for amplification means that the amplification reaction will be much more efficient. It is not yet clear whether covalent bonding of TP to the extremities of the DNA of ϕ29 is essential for amplification of this DNA, or whether the same system could be used to amplify heterologous DNA lacking TP bound to its extremities.

### DESCRIPTION OF THE INVENTION

The start of replication, based on templates with extremities of the DNA of ϕ29 lacking TP, is much less effective than the start of replication based on the ϕ29 genome as template, since the genome, along with the adequate extremities, contains TP covalently bound to the 5' phosphate of the extremities of both DNA strands.

The present invention describes something that has not been previously done and experimentally demonstrates *in vitro* DNA amplification, comparing that of the ϕ29 genome with TP covalently bound to its extremities (TP-DNA) versus other templates with terminal regions of the DNA of ϕ29, but without TP, using the minimum ϕ29 amplification machinery.

The authors of the present invention have found that in order for linear DNA to be amplified with the ϕ29 system, it must possess totally functional extremities of the DNA of ϕ29, distributed with the correct orientation and joined in a single DNA fragment.

The minimal origins of ϕ29 DNA replication described in this study do not require TP covalently bound to the 5' extremities in order to be functional in amplification, and can bind to the DNA destined for amplification using different common methods in molecular biology, such as ligation, recombination or cloning.

The *in vitro* DNA replication and amplification process has been studied for decades, and it has been necessary to experimentally perform each reaction to firmly establish the minimum requirements in order to ensure that such replication and amplification is functional.

The present study describes a method for the isothermal and defined amplification of large DNA fragments based on their insertion between two DNA sequences containing the minimum replication origins of the DNA of bacteriophage ϕ29. This method generates products possessing TP covalently bound to the 5' extremities of the amplified DNA, since TP is used as a "universal initiator" for all the amplifications made with this system.

The extremely high intrinsic processivity and capacity to couple polymerization to the band displacement of the DNA polymerase of ϕ29 make it possible to perform DNA amplifications of great length.

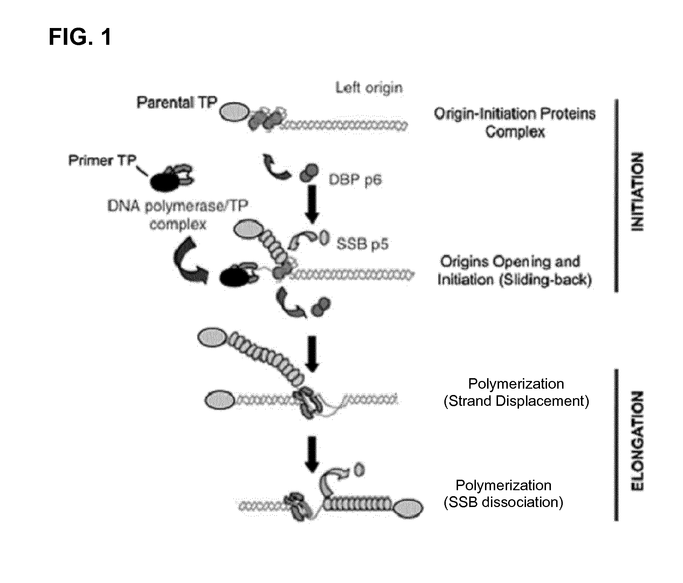

The resulting TP-DNA replicons can be used directly in applications in which the presence of TP is either of benefit or necessary, such as for example cell transformation, DNA transference or gene therapy *in vivo,* the *in vitro* evolution of proteins by DNA-display, bacterial transformation, sequencing, genotyping, etc. Alternatively, TP can be eliminated using different reactions known to experts in the field. Regarding paternal TP, it has been shown not to be absolutely essential for amplification. Paternal TP is the protein bound to the extremity of the parental DNA destined for replication or amplification. In order for replication or amplification to start, a new TP called the initiator TP must be incorporated (Figure 1).

Therefore, a first aspect of the present invention refers to a genic construct adapted for the introduction of an exogenous nucleotide sequence, and which consists of at least a nucleotide sequence with the following three elements in the stated order:
a) at least a nucleotide sequence comprising a replication origin of the DNA of bacteriophage ϕ29, with a terminal extremity followed by:
b) at least one cleavage site of the nucleotide sequence,
c) at least a nucleotide sequence comprising a replication origin of the DNA of bacteriophage ϕ29 with an orientation opposite that of the nucleotide sequence in (a).

The term "genic construct", as used in this study, refers to a DNA molecule that has been artificially assembled. The genic construct is adapted for the introduction of an exogenous nucleotide sequence, which may be any nucleotide sequence of interest.

The DNA of bacteriophage ϕ29 is linear and double stranded, and a replication origin is found at each of its extremities, referred to as left and right. The DNA replication origin of bacteriophage ϕ29 is the nucleotide sequence of one of the extremities of the genome of bacteriophage ϕ29, where replication of the mentioned genome starts. The terminal extremity of a replication origin is that where the TP binds.

A cleavage site is a region in the nucleotide sequence which as a result of its sequence is amenable to cleaving.

In a preferred approach to the first aspect of the invention, the nucleotide sequences of the three elements are juxtaposed or overlapped. It is more preferable for the nucleotide sequences of the three elements to be juxtaposed.

In a preferred approach to the first aspect of the invention, (b) is at least a nucleotide sequence comprising the cleavage target sequence of a restriction enzyme. Preferably, the restriction enzyme gives rise to blunt extremities or to protruding 3' extremities. More preferably, it gives rise to blunt extremities.

A cleavage site of a nucleotide sequence can have different presentations. In addition to cleavage with restriction enzymes, a nucleotide sequence can be cleaved with repair enzymes, or by generating an abasic site followed by treatment of the nucleotide sequence with an apurinic / apyrimidinic (AP) endonucleases. The nucleotide sequence can also be cleaved by introducing a ribonucleotide and then eliminating it with alkaline treatment. Another cleaving or cutting option is to introduce two cleavage sites in the nucleotide sequence, one in each strand, for action upon of enzymes that produce single-strand incisions.

The ϕ29 amplification system based on the vector pETORPHIBae (SEQ ID NO: 3), presented in the examples of the present report can effectively generate amplification products from templates with simple band extensions of 5 nucleotides at the 3' extremity.

In a preferred approach to the first aspect of the invention, the genic construct is a circular vector. The size of the genic construct is preferably between 4800 and 10,000 base pairs (bp).

A vector is a nucleic acid molecule used to transfer genetic material of interest to a cell. Apart from such genetic material, a vector can also contain different functional elements, including transcription control elements such as promoters or operators, transcription factor binding enhancers or regions, and control elements for starting and ending translation. Vectors include, but are not limited to the following:
plasmids, cosmids, viruses, phages, recombinant expression cassettes and transposons. Some vectors are able to replicate or divide autonomously once introduced in the host cell, such as bacterial vectors with a bacterial replication origin, or mammalian episomal vectors. Other vectors can become integrated within the host cell genome and thus replicate along with the cell genome.

In a preferred approach to the first aspect of the invention, the replication origins of the DNA of bacteriophage ϕ29 corresponding to the nucleotide sequences of (a) and (b) are of the same extremity of the DNA of bacteriophage ϕ29. The DNA extremity of bacteriophage ϕ29 is preferably the left extremity. The DNA extremity of bacteriophage ϕ29 is preferably the right extremity.

In a preferred approach to the first aspect of the invention, the replication origin of the DNA of bacteriophage ϕ29 corresponding to the nucleotide sequence (a) and the replication origin of the DNA of bacteriophage ϕ29 corresponding to the nucleotide sequence (c) are of different extremities of the DNA of bacteriophage ϕ29. Preferably, one replication origin of the DNA of bacteriophage ϕ29 is the left extremity, while the other replication origin of the DNA of bacteriophage ϕ29 is the right extremity.

In a preferred approach to the first aspect of the invention, the nucleotide sequence comprising the left replication origin of the DNA of bacteriophage ϕ29 contains between 65 and 200 nucleotides of the left extremity of the DNA of bacteriophage ϕ29. It preferably contains between 65 and 72 nucleotides of the left extremity of the DNA of bacteriophage ϕ29.

In a preferred approach to the first aspect of the invention, the nucleotide sequence comprising the right replication origin of the DNA of bacteriophage ϕ29 contains between 125 and 250 nucleotides of the right extremity of the DNA of bacteriophage ϕ29. It preferably contains between 150 and 200 nucleotides of the right extremity of the DNA of bacteriophage ϕ29.

In a preferred approach to the first aspect of the invention, the genic construct moreover includes at least one multiple cloning site (MCS).

The term multiple cloning site (MCS) refers to a small nucleotide sequence containing the target sequences of numerous restriction enzymes.

In a preferred approach to the first aspect of the invention, the genic construct moreover includes at least one marker. The marker preferably is a gene encoding for antibiotic resistance. The antibiotic in question is preferably ampicillin or kanamycin.

The term "marker", as it is used in this study, refers to a nucleotide sequence that encodes for a marker peptide or marker protein, allowing us to confirm that the vector has been transfected or transduced correctly, and that its sequences are correctly expressed. The marker may be a nucleotide sequence encoding for a fluorescent protein or a gene encoding for antibiotic resistance, used to select the cells that carry the vector.

In a preferred approach to the first aspect of the invention, the cleavage site is the cleavage target nucleotide sequence of the restriction enzyme Dra I or Bae I.

In a preferred approach to the first aspect of the invention, the genic construct comprises SEQ ID NO: 1, SEQ ID NO: 2, SEQ ID NO: 3 or a biologically equivalent variant.

A biologically equivalent variant of a nucleotide sequence is a nucleotide sequence possessing the same biological activity, i.e., comprising the same elements or equivalent elements with the same function.

A second aspect of this invention refers to utilization of the genic construct of the first aspect or approach to the invention for amplifying an exogenous nucleotide sequence.

It is important to distinguish between the replication of a nucleotide sequence and the amplification of a nucleotide sequence. In effect, while replication is a linear process, amplification is an exponential process; consequently, the number of DNA molecules obtained with an amplification reaction is far greater than that obtained with a replication reaction.

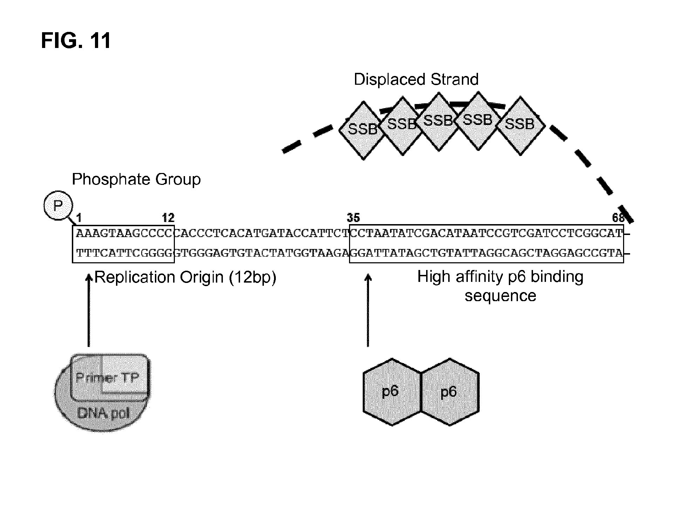

The authors of the present invention have demonstrated that it is possible to achieve efficient *in vitro* amplification by means of the DNA replication machinery of bacteriophage ϕ29, using as template a linear DNA with minimum replication origins of the DNA of ϕ29, but without TP covalently bound to the extremities. The DNA inserted between the origins of the DNA of ϕ29 can be heterologous. The minimum requirements of the *in vitro* system, as regards protein and DNA sequences, corresponding to the minimum origin of 68 bp of the left extremity of ϕ29, are shown in Fig. 11. As described by Gutiérrez et al. (Nucleic Acids Research 1988. Vol. 16 (13); 5895-5914), the last 12 bp of the extremities of the DNA of ϕ29 are needed to start replication, and for prolongation. This 12 bp DNA sequence is active as replication origin in the presence of TP and DNA polymerase, without the presence of any other protein; thus, mostly if not entirely, recognition of the origin must take place by means of the heterodimer TP-DNA polymerase. Although this complex is able to initiate replication with low efficiency, replication is greatly stimulated by the presence of p6. The minimum 68 bp origin of ϕ29 contains a high-affinity p6 binding site between nucleotides 35 and 68 with respect to the extremity. This nucleation site is necessary and sufficient for securing amplification stimulation mediated by this protein. Another essential protein for DNA amplification is SSB, which shows affinity for single strand DNA, without sequence specificity.

In a third aspect, this invention refers to a oligonucleotide comprising a nucleotide sequence of between 65 and 130 nucleotides from the left extremity of the DNA of bacteriophage ϕ29. It preferably contains between 67 and 72 nucleotides of the left extremity of the DNA of bacteriophage ϕ29. The 5' extremity of the oligonucleotide of this third aspect of the invention is preferably phosphorylated.

In a preferred approach to the third aspect of the invention, the oligonucleotide comprises the target nucleotide sequence of a restriction enzyme or the nucleotide sequence resulting from cleavage with a restriction enzyme in position 3' with respect to the nucleotide sequence including between 65 and 130 nucleotides or between 67 and 72 nucleotides of the left extremity of the DNA of bacteriophage ϕ29.

A fourth aspect of the present invention refers to utilization of the oligonucleotide of the third aspect of the invention for constructing a recombinant linear nucleotide sequence in which an exogenous nucleotide sequence destined for amplification is flanked by nucleotide sequences comprising the sequence including between 65 and 130 nucleotides or between 67 and 72 nucleotides of the left extremity of the DNA of bacteriophage ϕ29.

The authors of this invention have seen that the phosphate group at the 5' extremities of the replication origins is also essential for DNA amplification, especially for the elongation process. The addition of a small molecule, such as a propyl group, to the 5' phosphate impedes use of the origin for amplification.

A fifth aspect of the present invention refers to a DNA amplification method comprising at least the following steps:
a) obtainment of a linear DNA molecule comprising the DNA sequence destined for amplification and flanked on both extremities by:
   i) the sequence of the left replication origin of the DNA of bacteriophage ϕ29, the terminal extremity of this origin being located in the linear DNA extremity; or by
   ii) the sequence of the right replication origin of the DNA of bacteriophage ϕ29, the terminal extremity of this origin being located in the linear DNA extremity; or by
   iii) the sequence of the right replication origin of the DNA of bacteriophage ϕ29 on one side and of the left replication origin on the other - the terminal extremity of these origins being located in the two extremities of the linear DNA;
b) amplification of the linear DNA sequence obtained in step (a).

In a preferred approach to the fifth aspect of the invention, use is made of a DNA polymerase, a terminal protein (TP), a single strand DNA binding protein (SSB) and a double strand DNA binding protein (DBP) in the amplification of step (b). The proteins DNA polymerase, TP, p5 and p6 of bacteriophage ϕ29, or any bioequivalent variant of these proteins, are preferably used.

A bioequivalent variant of a protein or a biologically equivalent variant of a protein is a protein possessing the same biological activity, i.e., with the same function. In the present example, this may be DNA polymerase activity, primer activity in the case of TP, double strand DNA binding in the case of p6, or single strand DNA binding in the case of protein p5.

In a preferred approach to the fifth aspect of the invention, the DNA sequence destined for amplification is between 500 and 100,000 bp in size.

In a preferred approach to the fifth aspect of the invention, the linear DNA sequence obtained in step (a) presents phosphorylation of both 5' extremities.

In a preferred approach to the fifth aspect of the invention, the sequence of the left replication origin of the DNA of bacteriophage ϕ29 contains between 65 and 200 nucleotides of the left extremity of the DNA of bacteriophage ϕ29. It preferably contains between 67 and 72 nucleotides of the left extremity of the DNA of bacteriophage ϕ29.

In a preferred approach to the fifth aspect of the invention, the sequence of the right replication origin of the DNA of bacteriophage ϕ29 contains between 125 and 250 nucleotides of the right extremity of the DNA of bacteriophage ϕ29. It preferably contains between 150 and 200 nucleotides of the right extremity of the DNA of bacteriophage ϕ29.

In a preferred approach to the fifth aspect of the invention, the sequence of the left replication origin of the DNA of bacteriophage ϕ29 contains between 65 and 200 nucleotides or between 67 and 72 nucleotides of the left extremity of the DNA of bacteriophage ϕ29, and the sequence of the right replication origin of the DNA of bacteriophage ϕ29 contains between 125 and 250 nucleotides or between 150 and 200 nucleotides of the right extremity of the DNA of bacteriophage ϕ29.

In a preferred approach to the fifth aspect of the invention, step (b) is carried out at a temperature of under 30°C, preferably under 27°C, and even more preferably at a temperature of between 20-25°C.

In a preferred approach to the fifth aspect of the invention, step (a) makes use of the genic construct of the first aspect of the invention. In a preferred approach to the fifth aspect of the invention, step (a) makes use of the oligonucleotide of the third aspect of the invention.

A sixth aspect of the present invention refers to a kit for the amplification of an exogenous nucleotide sequence, consisting of at least a genic construct according to the first aspect of the invention, at least an oligonucleotide according to the third aspect of the invention, or both.

In a preferred approach to the sixth aspect of the invention, the genic construct comprises SEQ ID NO: 1, SEQ ID NO: 2 or SEQ ID NO: 3.

In a preferred approach to the sixth aspect of the invention, the kit moreover includes a DNA polymerase, a terminal protein (TP), a single strand DNA binding protein (SSB) and/or a double strand DNA binding protein (DBP). The DNA polymerase, the TP, the single strand DNA binding protein (SSB) and/or the double strand DNA binding protein (DBP) are preferably the DNA polymerase, TP, p5 and/or p6 of bacteriophage ϕ29, or a bioequivalent variant of these proteins.

In a preferred approach to the sixth aspect of the invention, the kit moreover comprises at least one of the elements of the following list: deoxynucleotide triphosphates (dNTPs), (NH₄)₂SO₄, MgCl₂, dithiothreitol (DTT), glycerol, bovine serum albumin (BSA) and Tris-HCl buffer with a pH of between 6.5 and 8.

A seventh aspect of this invention refers to utilization of the kit of the sixth aspect of the invention for amplifying an exogenous nucleotide sequence.

Throughout the description and the claims, the term "comprises" and its variants do not aim to exclude other technical characteristics, additives, components or steps. For the experts in the field, other objects, advantages and characteristics of the invention will be drawn in part from the description, and in part from implementation of the invention. The following examples are provided for illustrative purposes, and do not aim to impose limitations upon the present invention.

### DESCRIPTION OF THE FIGURES

**Figure 1****.** Schematic representation of the terminal protein (TP) - primed replication of the DNA of bacteriophage ϕ29. The initiator and paternal TP are shown in black and gray, respectively. The different replication phases are indicated to the right of the figure.

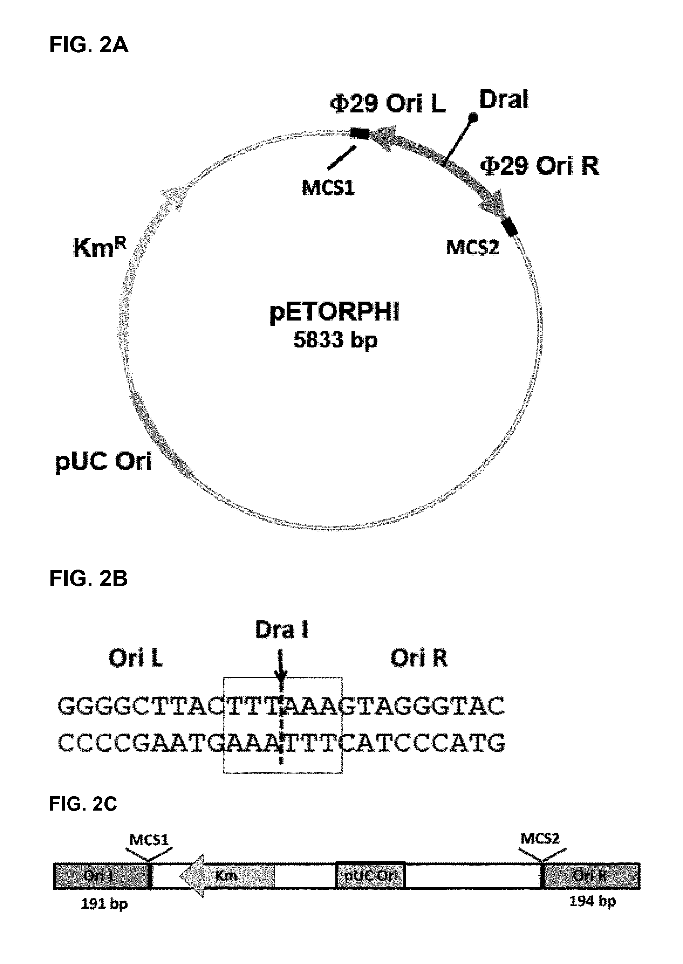

**Figure 2****.** Genic construct presenting the replication origins of the DNA of bacteriophage ϕ29. A. The pETORPHI plasmid was constructed by cloning a DNA fragment synthesized by Genescript, containing fused fragments of 194 base pairs (bp) and 191 bp from the left and right extremities of the genome of bacteriophage ϕ29, respectively, between sites Sac I and Hind III of pET28b. B. The extremities were inverted with an "extremity-to-extremity" orientation, generating a Dra I site at the joining point, as shown in the figure. "Ori L" and "Ori R" indicate the left and right origins of the DNA of ϕ29, respectively. C. The DNA fragment produced by plasmid linearization with Dra I, which presents the extremities of the DNA of ϕ29 correctly oriented. MCS1 and MCS 2 indicate the multiple cloning sites 1 and 2.

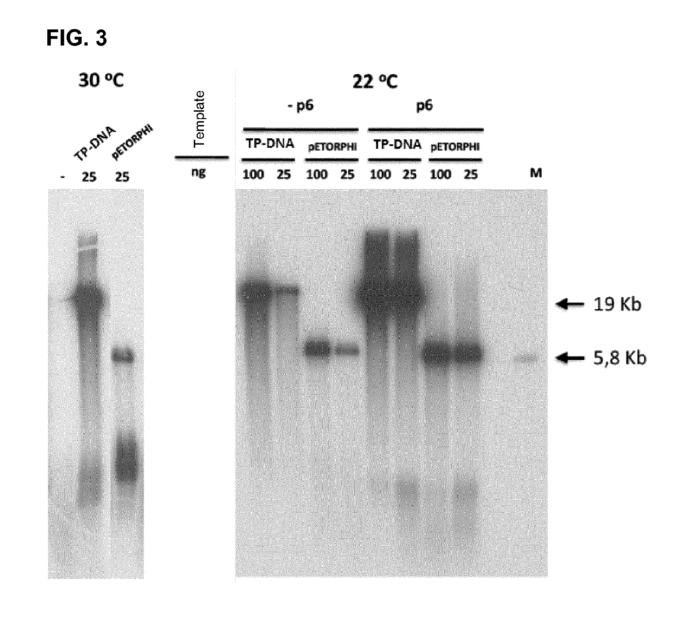

**Figure 3****.** The amplification of pETORPHI is more effective at 22°C. The DNA bands in agarose gel obtained in the amplification assays are shown. The amounts in nanograms (ng) of pETORPHI plasmid or TP-DNA templates of bacteriophage ϕ29 used in the amplification assays are shown. Both the incubation temperatures and the absence or presence of p6 are indicated. The reactions incubated at 30°C contained p6 in all cases. Use was made of 37.5 ng of TP and 25 ng of DNA polymerase. The size of the amplified bands is indicated: 19 kilobases (kb) for ϕ29 TP-DNA and 5.8 kb for pETORPHI cleaved with Dra I. The amplification factors calculated at 22°C were 80-fold for TP-DNA and 24-fold for pETORPHI.

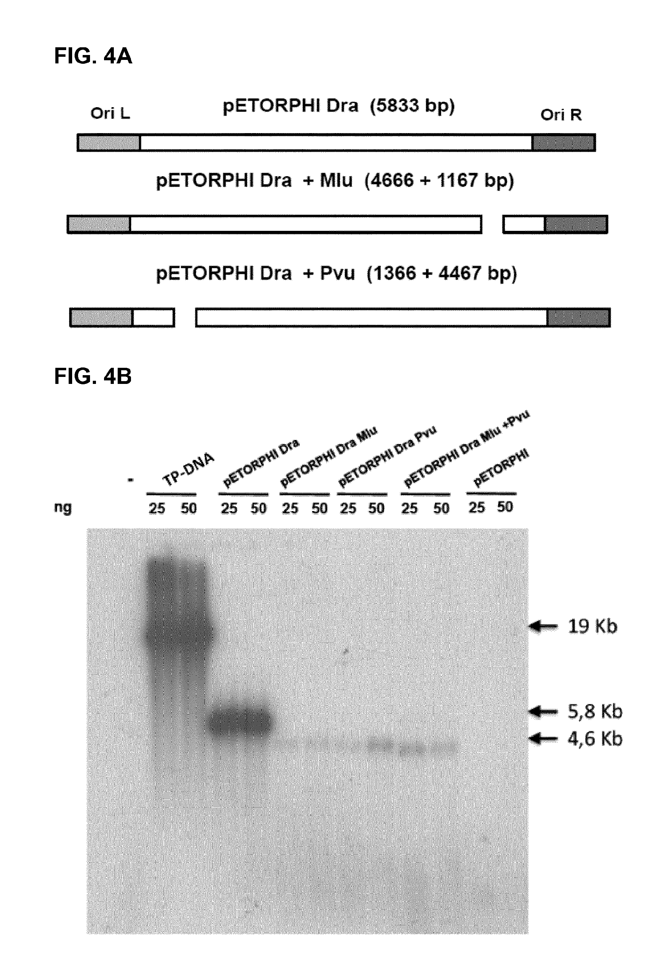

**Figure 4****.** Amplification requires two extremities of ϕ29 DNA in the same molecule. The pETORPHI plasmid was cleaved with the enzymes shown in the figure. A. Fragments resulting from the digestion of pETORPHI with the enzymes Dra I and Mlu I or Pvu I. B. DNA bands in agarose gel resulting from the amplification reaction using ϕ29 TP-DNA or the different plasmid fragments as template. The amounts (in ng) of ϕ29 TP-DNA or of fragments of the pETORPHI plasmid are shown. The bands corresponding to the plasmid cleaved with Mlu I or Pvu I, in addition to Dra I, are 4.6 and 4.4 kb in size, respectively. The amplification factors 109-fold for TP-DNA and 40-fold for pETORPHI. I.

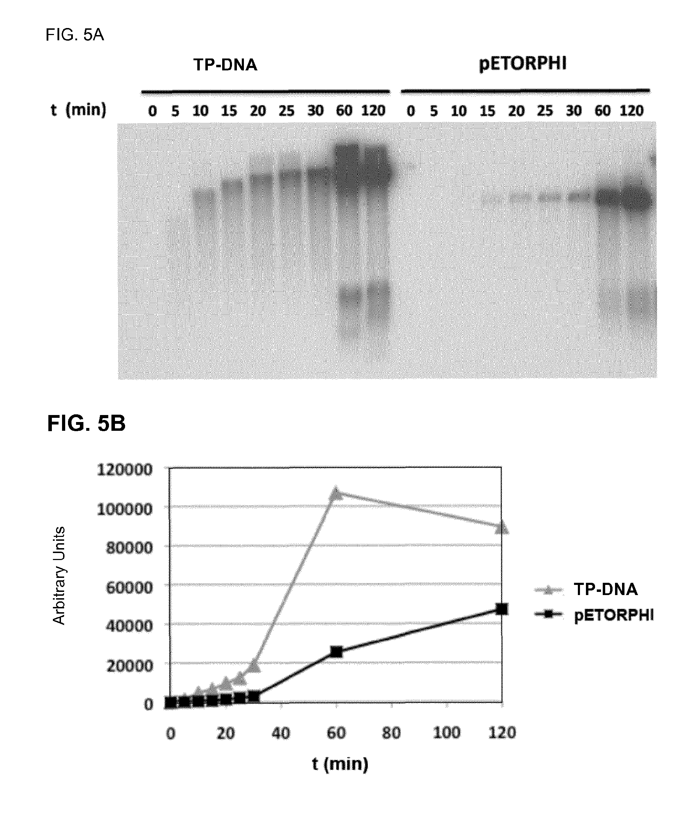

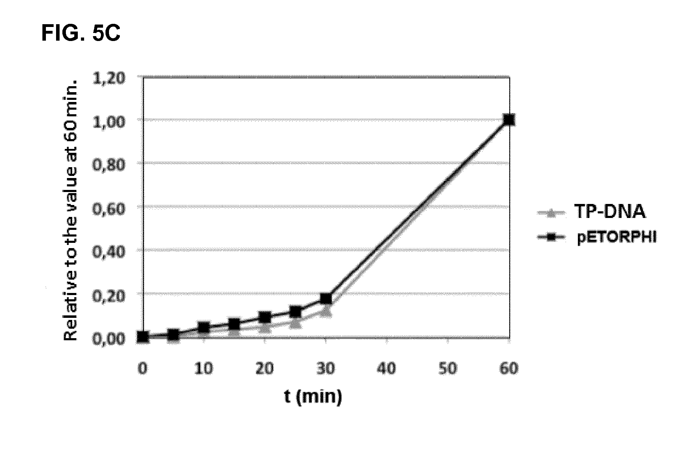

**Figure 5****.** The amplification kinetics of pETORPHI are similar to those of the genome of bacteriophage ϕ29. A. Products of the amplification reactions in agarose gel. These reactions contained 25 ng of ϕ29 TP-DNA or pETORPHI cleaved with Dra I; incubation was carried out at 22°C during the specified periods of time, and the reactions were stopped by adding 0.1% SDS and EDTA 10 mM. The amounts of TP and DNA polymerase were 37.5 and 25 ng, respectively. B. The gel bands of the experiments described in A were quantified using the Fosforimager system, and the values obtained were represented as a function of time. The absolute values are shown in section B. C. Representation of the values quantified in B, standardized with respect to the value at one hour (h). The amplification factors were 32-fold for TP-DNA and 14-fold for pETORPHI.

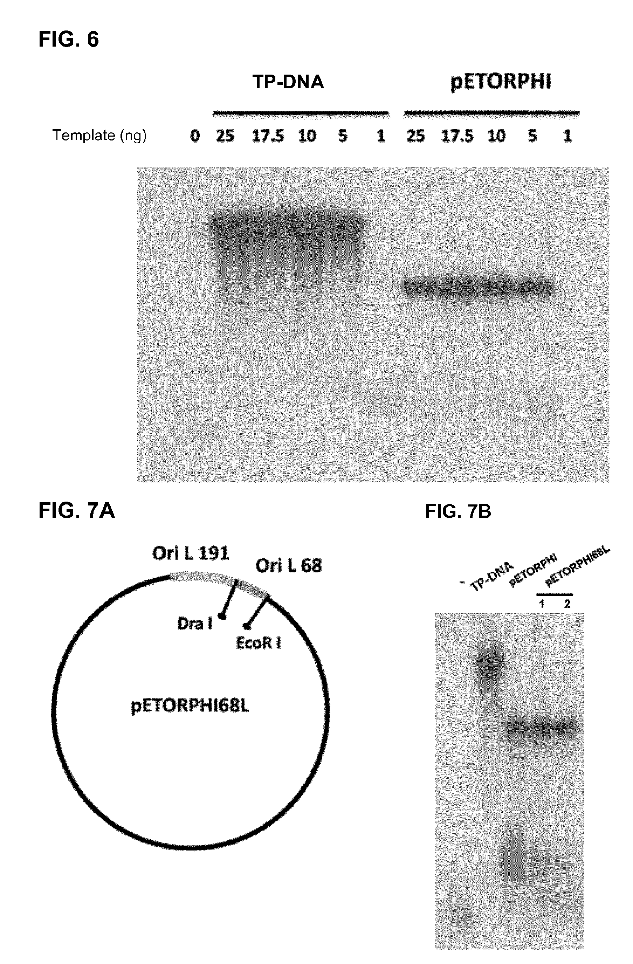

**Figure 6****.** The minimum amount of template needed for amplification is seen to be 5 ng. The amplification reactions were established with the indicated amounts (in ng) of ϕ29 TP-DNA or pETORPHI plasmid cleaved with Dra I, and incubated at 22°C during two hours. The amplification factors for 5 ng were 309-fold for TP-DNA and 141-fold for pETORPHI.
**Figure 7****.** A 68 bp fragment from the left extremity of the DNA of bacteriophage ϕ29 is seen to be an efficient amplification origin. A. The pETORPHI68L plasmid was constructed by cleaving pETORPHI with Dra I and EcoR I to eliminate the 191 bp fragment, corresponding to the right extremity of the genome of ϕ29, with replacement by a 68 bp fragment from the left extremity of the genome of ϕ29 (shown at left). B. For the amplification reaction, pETORPHI68L was cleaved with Dra I, and the reaction was incubated at 22°C with 25 ng of the corresponding template. Numbers 1 and 2 of the lanes with pETORPHI68L correspond to two different clones of this plasmid. The negative control in the absence of template is indicated as -. TP-DNA and pETORPHI were used as positive controls.

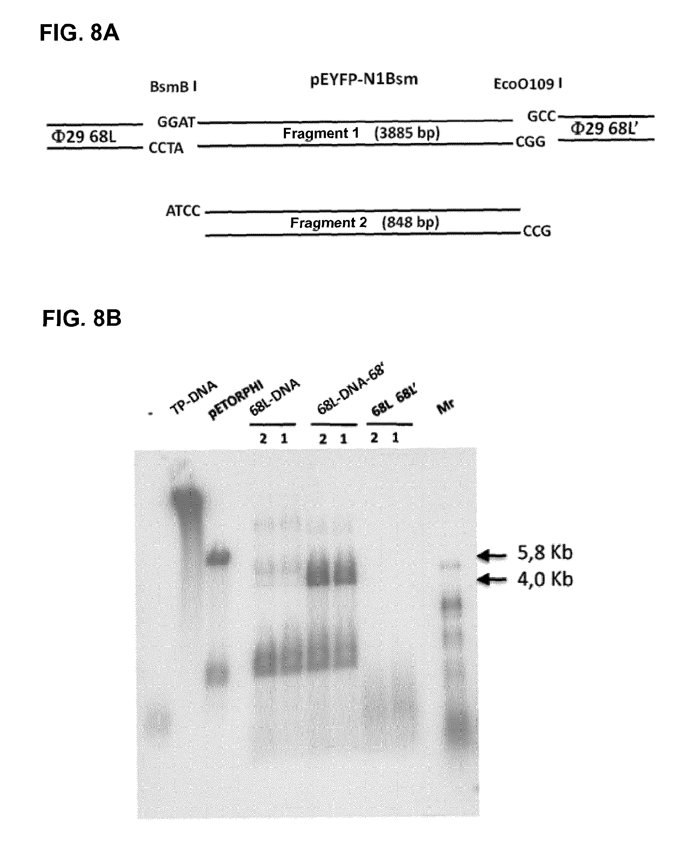

**Figure 8****.** Ligation of a 68 bp DNA fragment from the left extremity of the genome of bacteriophage ϕ29 to the two extremities of a heterologous DNA fragment allows amplification of the latter. A. DNA fragments corresponding to the 68 bp origin of the left extremity of the genome of ϕ29 were assembled, using single strand oligonucleotides that generated cohesive extremities for enzymes BsmB I (indicated as 68L) and EcoO109 I (indicated as 68L'). The pEYFP-N1Bsm plasmid was cleaved with BsmB I and EcoO109 I, and the fragments of this plasmid were included in a ligation reaction with the described oligonucleotides 68L and 68L'. B. Amplification requires the presence of the extremities of the genome of Φ29 at both extremities of the heterologous DNA, since amplification is only observed when the template is the product of ligation of the heterologous DNA with nucleotides 68L and 68L'. Ligation was carried out either with oligonucleotides 68L and 68L' and fragment 1 of the pEYFP-N1Bsm plasmid (lanes 1 and 2 indicated as 68L-DNA-68L') or with only one of the nucleotides plus fragment 1 (lanes 1 and 2 indicated as 68L-DNA), and also in the absence of fragment 1 and with the two types of nucleotides, as control (lanes 1 and 2 indicated as 68L and 68L'). In the amplification reactions, aliquots of the ligations were used as templates; accordingly, each reaction contained the same number of moles for both the fragment pertaining to the plasmid (4 kb) and for pETORPHI.

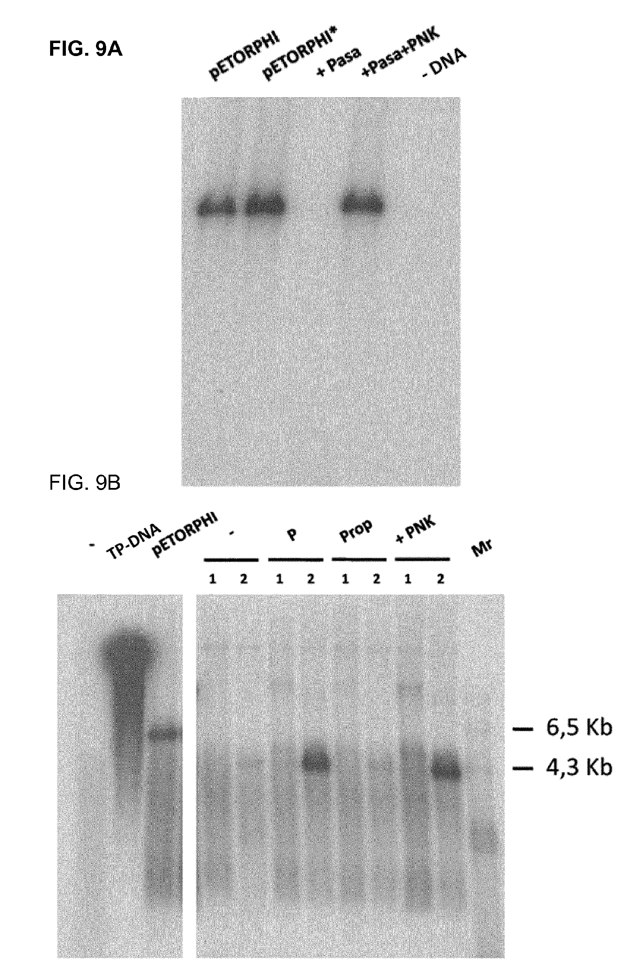

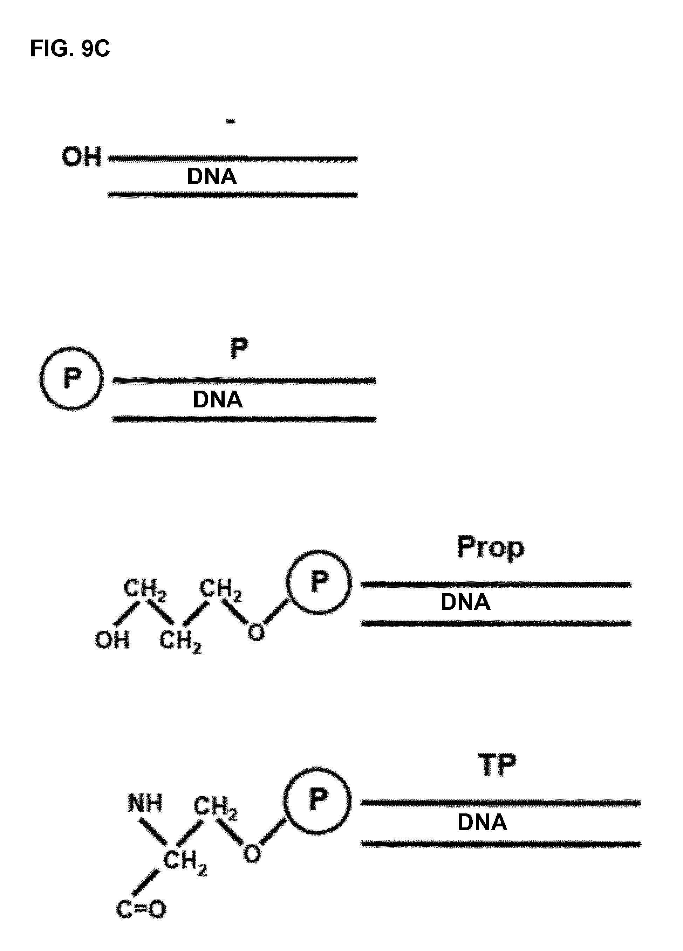

**Figure 9****.** The phosphate group in position '5 is necessary for amplification. A. The DNA bands obtained in the amplification reactions of aliquots of pETORPHI treated with Antarctic phosphatase (+Pase), with adenosine triphosphate (ATP) and T4 polynucleotide kinase, after phosphatase (+Pase +PNK), or subjected to the same processing as the treated samples, but without adding enzymes (pETORPHI*). In all cases, the amount of pETORPHI present in the reactions was 25 ng. A reaction in the absence of DNA template was used as negative control. B. The same ligations as those described in Figure 8B were performed. In addition, the oligonucleotides were designed to present no modification at their 5' extremities 5' (-), phosphorylation at 5' (P), or propyl group bound to the phosphate at 5' (Prop), or unmodified oligonucleotides were treated with ATP and T4 polynucleotide kinase after ligation (+PNK). Lanes 1 indicate that only oligonucleotide 68L was used in ligation, and lanes 2 indicate that both oligonucleotides (68L and 68L') were used. C. Schematic representation of the chemical structures located at extremity 5' of the oligonucleotides: unmodified (-), modified with a phosphate group (P), modified with a propyl group bound to the phosphate (Prop), and bound to the serine group of TP, through the phosphate group.

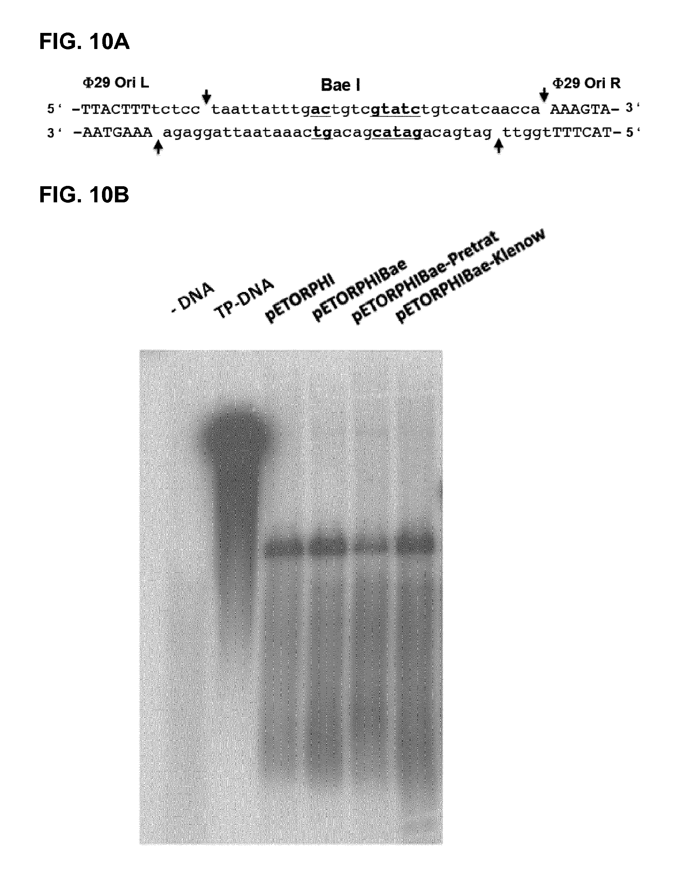

**Figure 10****.** A pETORPHI derivative called pETORPHIBae, linearized with Bae I is seen to be effective as a template for amplification. A. Schematic representation of the recognition site of Bae I (underlined) and of the cleaving points (arrows) and positions of the origins of ϕ29 (capital letters) in pETORPHIBae. B. The DNA bands resulting from the amplification reactions using TP-DNA and pETORPHI as positive controls, the absence of DNA as negative control (-), and the pETORPHIBae plasmid linearized with Bae I without modifications, and with different treatments indicated as "Pretrat" and "Klenow". "Pretrat" indicates that pETORPHIBae linearized with Bae I was pretreated for 5 minutes (min) with the DNA polymerase of ϕ29, after which the rest of the components of the reaction were added, except TP, which was added 5 min later. "Klenow" indicates that pETORPHIBae linearized with Bae I was treated for 10 min with the Klenow enzyme in the presence of deoxyribonucleoside triphosphates (dNTPs); the mentioned enzyme was then deactivated, and the rest of the components of the reaction were added. Aliquots of 25 ng of pETORPHIBae linearized with Bae I were used in the three amplification reactions.
**Figure 11****.** The requirements of the origins of the DNA of bacteriophage ϕ29 for *in vitro* amplification. The origin of the 68 bp left extremity of ϕ29 DNA is shown. The 12 bp located on the same extremity (rectangle) are necessary for the initiation and elongation reactions performed by the DNA polymerase of ϕ29, forming a heterodimer composed of DNA polymerase (represented as an oval) bound to TP as initiator (Init. represented in the form of a rectangle). The phosphate group, located at extremity 5' (P), was seen to be essential for *in vitro* amplification. Protein p6 (hexagon) and the high affinity sequences (rectangle between positions 35 bp and 68 bp) are necessary for stimulating initiation by this protein. Amplification requires SSB (rhombus) to bind to the displaced DNA strand, represented as a broken line.

### EXAMPLES

The invention is illustrated below by means of a series of assays made by the inventors, demonstrating the efficacy of the amplification method of the invention, as well as of the nucleotide sequences used to develop the technique.

### EXAMPLE 1: Design of a plasmid generating the functional extremities of the DNA of bacteriophage ϕ29 when linearized.

We designed the plasmid described in Figure 2A, called pETORPHI (SEQ ID NO: 1). The pETORPHI plasmid was constructed by cloning between sites Sac I and Hind III of pET28b (SEQ ID NO: 11) a DNA fragment synthesized by Genescript, containing fused fragments of 194 base pairs (bp) and 191 bp from the left and right extremities of the genome of bacteriophage ϕ29, respectively. The extremities were inverted with an "extremity-to-extremity" orientation, generating a single Dra I restriction site at the joining point, as shown in Figure 2B. The pETORPHI possesses a backbone that contains no Dra I restriction site, a multiple cloning site (MCS), a pUC type replication origin (Ori), a resistance marker consisting of a kanamycin resistance gene, and the right and left extremity sequences of the DNA of the genome of bacteriophage ϕ29, reaching base pairs (bp) 191 and 194 from the extremity, respectively (SEQ ID NO: 12 and SEQ ID NO. 13, respectively). After cleaving the plasmid with Dra I, the two extremities of the linearized plasmid are identical to the two extremities of the DNA of ϕ29 up to the aforementioned positions (Figure 2C).

### EXAMPLE 2: Amplification using the linearized pETORPHI plasmid as template.

We checked the capacity of the pETORPHI plasmid cleaved with Dra I (henceforth referred to as pETORPHI) to act as a template in amplification started with terminal protein (TP) by means of the proteins DNA polymerase, p5 and p6 of bacteriophage ϕ29. As can be seen in Figure 3, at left, on incubating the amplification reaction at 30°C during two hours and using the genome of ϕ29 with TP bound to its 5' extremities, we obtain an amplified product in the expected position. Lesser amounts of the correct product (5.8 kb) are obtained on using pETORPHI as template, and a larger amount of material of lesser molecular weight is produced. Surprisingly, when the amplification reaction is carried out at 22°C, the presence of these products of lesser molecular weight was found to decrease. In the absence of p6 there is a certain amount of correct amplified products, though the reaction does not reach maximum performance - not even starting with 100 ng of genome of bacteriophage ϕ29 or plasmid as template. In the presence of p6, the reaction reaches a plateau with 25 ng of ϕ29 genome or with the same amount of pETORPHI, and most of the amplified DNA is moreover in the correct position, with almost no lesser molecular weight products. All the subsequent experiments were made at 22°C. DNA band quantification was carried out with the Fosforimager system. The calculated amplification factors are 80-fold for the bacteriophage genome and 24-fold for the pETORPHI plasmid.

In order to determine whether the products were amplification products or linear replication products, the origins of the DNA of ϕ29 of the pETORPHI plasmid were separated into two molecules, so that both DNA fragments would not be susceptible to exponential amplification, since each DNA molecule would only contain one of the ϕ29 replication origins - thereby giving rise to linear replication. We chose two enzymes, Mlu I and Pvu I, which each cleave pETORPHI once, giving rise to a series of fragments of about 4.7 and 1.2 Kb, and 1.4 and 4.5 Kb in size, respectively, on using these enzymes with Dra I, as can be seen in Figure 4A. Aliquots of the linearized pETORPHI plasmid were treated using Dra I, with either Mlu I or Pvu I, or with restriction buffer as control, using them as templates in the amplification reaction. Figure 4B shows that in all cases in which we separate the two extremities of the pETORPHI plasmid, the amount of amplified DNA decreases drastically (about 30-fold) when compared with the signal obtained using pETORPHI. Even when the fragments are cleaved with the two enzymes independently and are mixed in the amplification reaction to check whether they hybridize or complement each other (Figure 4A, lanes Dra Mlu +Pvu), the results are almost identical to those obtained with fragments cleaved with only one enzyme. The non-cleaved pETORPHI plasmid produces no signal. Likewise, it was checked that the different templates had active origins referred to the start of replication, to ensure that the amplification defects are only due to separation of the two origins into two molecules.

The results indicate that the pETORPHI plasmid gives rise to true amplification, using the origins located at both extremities of the molecule.

### EXAMPLE 3: Amplification kinetics.

Amplification experiments were carried out, detecting the products over time and comparing the results obtained using the genome of bacteriophage ϕ29 or pETORPHI as template. Figure 5A shows that both templates yield a very similar profile, with small amounts of products generated up to 30 minutes (min), followed by a gradual increase to a maximum reached after 60 min with the genome of ϕ29, or one-half of the maximum with pETORPHI. Having reached this point, pETORPHI continues to generate products for another 60 min. Quantification using the Fosforimager system showed the amount of synthesized TP-DNA to increase up to 60 min, where the TP-DNA amplification saturation point is reached (Figure 5B). In the case of the pETORPHI vector, amplification continues until saturation is reached after 120 min. The values obtained up to 60 min, standardized by taking the value at 60 min as equal to 1, are practically identical for the genome of ϕ29 and pETORPHI (Figure 5C). This indicates that up until 60 min, the amplification reaction proceeds in a very similar manner for both TP-DNA of ϕ29 and pETORPHI, with the difference that saturation has already been reached after 60 min with TP-DNA, while pETORPHI continues to generate amplification product for an additional hour. Longer incubation times of up to 5 hours (h) did not imply an increase in total product generated with either TP-DNA of ϕ29 or pETORPHI.

### EXAMPLE 4: Minimum amount of template required for amplification.

We determined the minimum amount of template required for amplification. Figure 6 shows that on using the genome of ϕ29 as template, the minimum amount allowing amplification is 5 ng, and the amount of product obtained is very similar when using 10 to 25 ng of template. This indicates that amplification is close to the saturation point in that template concentration interval. No amplification is obtained with 1 ng. The results obtained on using pETORPHI as template are similar. In this case 3.3 times more template molecules were used than with the genome of ϕ29. It is important to point out that on starting the reaction, pETORPHI does not have the paternal TP, while the genome of ϕ29 does have the paternal TP, and the latter increases the affinity of the extremities with respect to the replication machinery.

### EXAMPLE 5: Minimum replication origin.

The minimum length of DNA of bacteriophage ϕ29 allowing replication activity stimulated with p6 corresponds to the last 68 bp of the left extremity of the genome of ϕ29. This includes the inverted repetition of 6 bp (5' AAAGTA 3') and the sequences of 35 to 68 bp of the left extremity that constitute high affinity sites for the binding of p6.

The 194 bp segment corresponding to the right extremity of the genome of ϕ29 of pETORPHI was replaced by the 68 bp minimum replication origin of the left extremity, described above, to obtain the so-called pETORPHI68L (SEQ ID NO: 2) (Figure 7A).

The 68 bp fragment was cloned with an inverted orientation, regenerating the Dra I restriction site as in pETORPHI, in order to obtain the DNA extremities of ϕ29 when pETORPHI68L is linearized with Dra I. Figure 7B shows that the capacity of pETORPHI68L to act as an amplification template is the same as that of the original pETORPHI.

### EXAMPLE 6: Amplification after ligation with oligonucleotides that include the minimum origins of the DNA of ϕ29.

After confirming that a 68 bp fragment of the left extremity of the DNA of bacteriophage ϕ29 is an effective amplification origin, we can design oligonucleotides which following hybridization generate the 68 bp fragment as double strand DNA to which we can add the sequence of interest, such as for example single strand DNA extensions to ligate them to extremities of exogenous DNA obtained after digestion with restriction enzymes. By means of this process we can generate oligonucleotides of ϕ29 DNA with which the minimum replication origin of ϕ29 can bind to any linear DNA chosen for amplification.

Using guided mutagenesis, we introduced the sequence of the target of restriction enzyme BsmB I in the pEYFP-N1 plasmid (SEQ ID NO: 14) (Clontech) in position 4702 to generate the pEYFP-N1Bsm plasmid (SEQ ID NO: 15). Posteriorly, pEYFP-N1Bsm was cleaved with the enzymes BsmB I and EcoO109 I, which recognize single sequences in that vector. Likewise, we used two pairs of synthetic oligonucleotides (SEQ ID NO: 7 and SEQ ID NO: 8), whereby after hybridization, each pair would form the 68 bp double strand DNA corresponding to the minimum origin of ϕ29, in addition to short single strand extensions allowing ligation to one or other of the extremities obtained on cleaving the pEYFP-N1 Bsm plasmid with enzyme BsmBI or EcoO109I, respectively (Figure 8A). We chose these enzymes because they generate asymmetrical extremities, thereby avoiding the auto-ligation of oligonucleotides that contain the same cohesive extremity, as well as cross-ligation between sites BsmB I and EcoO109 I. In this way, following hybridization, each pair of oligonucleotides can ligate to the complementary restriction extremity of the plasmid, but not to the other extremity, or to the other pair of oligonucleotides, and likewise cannot undergo auto-ligation. On the other hand, this restriction based approach makes it unnecessary to carry out gel purification of the DNA fragments or ligation products. Following the ligation reaction, the ligase was inactivated and the sample was treated with adenosine triphosphate (ATP) and T4 polynucleotide kinase.

Utilization of the DNA resulting from the ligation reactions as templates only allows real amplification of one of the ligation products, namely the 3885 bp fragment of the pEYFP-N1Bsm plasmid (Figure 8A, fragment 1), ligated to the two oligonucleotides with sequences that comprise the 68 bp of the left extremity of the DNA of bacteriophage ϕ29. This product generates a single 4021 bp band corresponding to the scheme: ϕ29 68L- BsmBI-pEYFP-N1 Fragment 1-EcoO109I-ϕ29 68L.

The other fragment (Fig. 8A, fragment 2) does not ligate to the oligonucleotides, and is therefore not amplified. As can be seen in Figure 8B, only ligation comprising all the adequate components generates an amplified band of the appropriate length, while the control reactions performed with ligation mixture in the absence of oligonucleotides (68L-DNA), or containing only the two oligonucleotides (68L and 68L', without plasmid fragment), did not produce the correct amplified product. After quantification with the Fosforimager system, we calculated that the amplified band obtained with the ligation product corresponded to 50% of the molecules present in the pETORPHI band. The results confirm that it is possible to ligate minimum origins of DNA of bacteriophage ϕ29 to heterologous DNA to obtain TP-DNA of adequate length by means of the replication system of ϕ29, without the need for gel purification or cloning.

### EXAMPLE 7: The phosphate group at the 5' extremities of the DNA is necessary for amplification.

As described above, during the experiments carried out with oligonucleotides containing the minimum origins of ϕ29, we found that the extremities of the DNA of bacteriophage ϕ29 containing non-phosphorylated oligonucleotides at the 5' extremity could not be used as amplification template. Therefore, we treated the ligation products with ATP and T4 polynucleotide kinase, after which amplification of the templates obtained by ligation of the abovementioned oligonucleotides was effectively observed. In the above section, in order to avoid ligation of the extremities of the DNA of ϕ29 with themselves through their blunt extremities, the oligonucleotides which we used were previously dephosphorylated and, after ligation, were treated with ATP and T4 polynucleotide kinase. In order to confirm the importance of the 5' phosphate group in the amplification process, we carried out the experiment shown in Figure 9A. An aliquot portion of pETORPHI was cleaved with Dra I and treated with Antarctic phosphatase (New England Biolabs). Then, one half of the sample was treated with ATP and T4 polynucleotide kinase, while the other half was treated only with the kinase buffer, as control. After precipitating the samples and subjecting them to the amplification protocol, we found that the sample treated only with phosphatase yielded no amplification product. In contrast, both pETORPHI control and pETORPHI treated with kinase after phosphatase yielded the typical amount of amplified product after using pETORPHI as template (Figure 9).

In order to explore the selectivity of the replication machinery for the 5' extremities of the replication origins of ϕ29, we synthesized three oligonucleotides with the sequence of the 68 bp left origin of ϕ29 (SEQ ID NO: 5) and different modifications of the 5' extremity. Of the three oligonucleotides, one was not phosphorylated, another was phosphorylated at the 5' extremity, and the third received a propyl group added through the 5' phosphate. This latter oligonucleotide was designed to imitate the chemical structure bound to the 5' phosphate when TP is covalently bound to the DNA (Figure 9C). Following hybridization to form the 68 bp left replication origin, these oligonucleotides were ligated to Fragment 1 of the pEYFP-N1 plasmid (Figure 8A), and we checked the capacity of the resulting products to act as amplification template. The results shown in Figure 9B again demonstrate that the DNA generated after ligation of the dephosphorylated oligonucleotide is not a good template for amplification (lane -); in contrast, the DNA ligated to the phosphorylated oligonucleotide (lane P) or DNA phosphorylated after ligation (lane + PNK) constitute functional templates. On the other hand, the oligonucleotide synthesized with a propyl group bound to the 5' phosphate (lane Prop) did not give rise to amplification. These results show that we can use oligonucleotides with phosphorylated replication origins of ϕ29 for ligation to heterologous DNA, thereby yielding amplification of the global construct. In this context, an important requirement for amplification is to ensure that the 5' extremities of the origins are phosphorylated.

### EXAMPLE 8: Linearization of pETORPHI with enzyme Bae I.

We prepared a pETORPHI derivative called pETORPHIBae (SEQ ID NO: 3), in which a Bae I restriction site was inserted precisely at the junction of the extremities of the DNA of bacteriophage ϕ29, thereby eliminating the Dra I site. Bae I cleaves double strand DNA at two different points separated by 33 bp; presents its recognition site approximately half way between them; and cleaves DNA independently of the sequence located at the corresponding digestion points. Cleavage with Bae I leaves 5-nucleotide single band extensions at the two resulting 3' extremities (Figure 10A). In order to assess the effect of the extensions at the 3' extremities of the replication origins of ϕ29 upon amplification efficiency, we performed amplification using three different approaches: (i) amplification reaction under standard conditions after treatment with Bae I (lane pETORPHIBae); (ii) pretreatment of Bae I-cleaved pETORPHIBae with the DNA polymerase of ϕ29 in the presence of dNTPs, in order to eliminate the 3' extensions, followed by the addition of TP and the rest of the reaction components; and (iii) pretreatment with Klenow DNA polymerase and nucleotides during 10 min, likewise to produce blunt extremities, followed by addition of the components for carrying out a standard amplification reaction. As can be seen in Figure 10B, approaches (i) and (iii) give rise to amplification very similar to that obtained with pETORPHI, while pretreatment with the DNA polymerase of ϕ29 yields poorer results - possibly due to the great activity of the exonuclease of this enzyme.

### EXAMPLE 9: Genic constructs.

Genescript synthesized a DNA fragment corresponding to the extremities of the DNA of bacteriophage ϕ29 up to positions 194 and 191 bp, counting from the left and right extremities, respectively, of the genome of ϕ29. The fragment was designed with the extremities of the DNA of ϕ29 fused in an opposite orientation and forming a Dra I restriction site. The fragment was obtained cloned in the pUC57 plasmid (pUC57ORPHI) (SEQ ID NO: 4). The pETORPHI plasmid (SEQ ID NO: 1) was constructed by cloning a DNA fragment extracted from pUC57ORPHI, using the same restriction enzymes, between sites Sac I and Hind III of pET28b (SEQ ID NO: 11). In this way we obtained a single Dra I site in pETORPHI, and this site was located precisely at the junction between the replication origins of ϕ29.

In order to construct pETORPHI68L (SEQ ID NO: 2), we designed oligonucleotides which after hybridization conformed the last 68 bp of the left extremity of the DNA of bacteriophage ϕ29, in addition to an AATT extension to allow ligation to EcoR I sites (SEQ ID NO: 6).

pETORPHI was digested with Dra I and EcoR I in order to eliminate the 191 bp fragment corresponding to the right extremity of the DNA of ϕ29 from the vector, and the 68 bp fragment of the left extremity of the genome was cloned to replace the eliminated fragment. pETORPHIBae (SEQ ID NO: 3) was created by cleaving pETORPHI with Dra I and inserting a double strand phosphorylated oligonucleotide possessing a recognition site for Bae I (SEQ ID NO: 9 as direct strand and SEQ ID NO: 10 as reverse strand). In this way the cleavage site for that enzyme is located in a position immediately adjacent to the terminal extremities of the origins of ϕ29 in the 5' strand, while a 5-nucleotide extremity is left in the 3' strand (Figure 10A).

### EXAMPLE 10: Enzymatic digestions, ligation reactions and dephosphorylation.

Digestion of the vectors with the enzyme Dra I was carried out normally during four hours, using buffer 4 of New England Biolabs, except in the cases requiring multiple digestions with the enzymes Dra I, Mlu I and/or Pvu I; in these cases we used buffer 3 of New England Biolabs and 0.1 mg/ml of BSA.

Digestion with Bae I was carried out with buffer 4 of New England Biolabs, to which 0.1 mg/ml of BSA and 20 µM end concentration of S-adenosylmethionine were added.

All the enzymes were inactivated after the reactions by incubation at 65°C or 80°C, depending on the enzyme. In all cases the restriction cleavages were analyzed by agarose gel electrophoresis. We included control points without DNA but with the corresponding restriction buffers in all the amplification experiments.

In the experiments in which ligation proved necessary, the pEYFP-N1 plasmid was cleaved using BsmB I and EcoO109 I with buffer 3 of New England Biolabs during 8 hours, followed by purification using Qiagen columns and ligation in 1:4 proportion together with the hybridized oligonucleotides, in order to generate double strand DNA. After inactivating the ligase by heating to 65°C during 20 min, the samples were used as amplification reaction templates with no further treatment, except were indicated otherwise.

Dephosphorylation was carried out with Antarctic phosphatase from New England Biolabs, adding its corresponding buffer and incubating at 37°C for 15 min, followed by inactivation through incubation at 65°C during 20 min. Following this treatment, the samples were precipitated with ethanol in the presence of 20 µg of glycogen as transporter, to eliminate the phosphatase buffer. After precipitation of the samples, the latter were resuspended in ligase buffer and used directly in the amplification experiments. Where indicated, the samples were treated with ATP and T4 polynucleotide kinase from New England Biolabs, in T4 ligase buffer at 37°C during 30 min, and this enzyme was likewise inactivated through incubation at 65°C during 20 min.

### EXAMPLE 11: Amplification experiment.

The amplification reactions were carried out in a volume of 25 µl, in A 1X buffer (50 mM Tris-HCl, pH 7.5, 10 mM MgCl₂, 5% glycerol, 1 mM DTT, 0.1 mg/ml BSA) supplemented with (NH4)₂SO₄ to 20 mM and with an end concentration of 100 µM corresponding to each of the nucleotides: dCTP, dGTP, dTTP and dATP, [α-32P]dATP (1 µCi), 15 µg ϕ29 SSB (40 µM), 10 µg p6 (27 µM), 20 ng of DNA polymerase of bacteriophage Φ29 (13 nM) and 20 ng of Φ29 TP (26 nM), except where indicated otherwise, and the indicated amounts of vector or TP-DNA as template.

The reactions were started by adding the rest of the components to the template and TP (previously mixed), and were allowed to run for two hours at 22°C. The reactions were subsequently stopped by adding EDTA 10 mM and 0.1% SDS, as end concentrations. The samples were dried, resuspended in 50 µl of NaOH 0.5 M, and analyzed by 0.7% agarose gel electrophoresis in the presence of NaOH. We show the typical results of at least three independent experiments.

## Claims

1. A genic construct adapted for the introduction of an exogenous nucleotide sequence, and which consists of at least a nucleotide sequence with the following three elements in the stated order:
a) at least a nucleotide sequence comprising a replication origin of the DNA of bacteriophage ϕ29, with a terminal extremity followed by:
b) at least one cleavage point of the nucleotide sequence,
c) at least a nucleotide sequence comprising a replication origin of the DNA of bacteriophage ϕ29 with an orientation opposite that of the nucleotide sequence in (a).

2. The genic construct according to the above claim, where the nucleotide sequences of the three elements are juxtaposed or overlapped.

3. The genic construct according to any of the above claims, where the nucleotide sequences of the three elements are juxtaposed.

4. The genic construct according to any of the above claims, where (b) is at least a nucleotide sequence comprising the cleavage target sequence of a restriction enzyme.

5. The genic construct according to the above claim, where the restriction enzyme gives rise to blunt extremities or to protruding 3' extremities.

6. The genic construct according to any of the above claims, where the genic construct is a circular vector.

7. The genic construct according to any of the above claims, where the size is between 4800 and 10,000 base pairs (bp).

8. The genic construct according to any of the above claims, where the replication origin of the DNA of bacteriophage ϕ29 corresponding to the nucleotide sequences of (a) and (b) are of the same extremity of the DNA of bacteriophage ϕ29.

9. The genic construct according to the above claim, where the DNA extremity of bacteriophage ϕ29 is the left extremity.

10. The genic construct according to claim 6, where the DNA extremity of bacteriophage ϕ29 is the right extremity.

11. The genic construct according to any of the claims 1 to 7, where the replication origin of the DNA of bacteriophage ϕ29 corresponding to the nucleotide sequence (a) and the replication origin of the DNA of bacteriophage ϕ29 corresponding to the nucleotide sequence (c) are of different extremities of the DNA of bacteriophage ϕ29.

12. The genic construct according to the above claim, where one replication origin of the DNA of bacteriophage ϕ29 is the left extremity, while the other replication origin of the DNA of bacteriophage ϕ29 is the right extremity.

13. The genic construct according to any of the above claims 1 to 9, 11 or 12, where the nucleotide sequence comprising the left replication origin of the DNA of bacteriophage ϕ29 contains between 65 and 200 nucleotides of the left extremity of the DNA of bacteriophage ϕ29.

14. The genic construct according to any of the above claim, where the nucleotide sequence comprising the left replication origin of the DNA of bacteriophage ϕ29 contains between 65 and 72 nucleotides of the left extremity of the DNA of bacteriophage ϕ29.

15. The genic construct according to any of the above claims 1 to 8 or 10 to 12, where the nucleotide sequence comprising the right replication origin of the DNA of bacteriophage ϕ29 contains between 125 and 250 nucleotides of the right extremity of the DNA of bacteriophage ϕ29.

16. The genic construct according to the above claim, where the nucleotide sequence comprising the right replication origin of the DNA of bacteriophage ϕ29 contains between 150 and 200 nucleotides of the right extremity of the DNA of bacteriophage ϕ29.

17. The genic construct according to any of the above claims, which moreover comprises at least one multiple cloning site (MCS).

18. The genic construct according to any of the above claims, which moreover comprises at least one marker.

19. The genic construct according to the above claim, where the marker is an antibiotic resistance gene.

20. The genic construct according to the above claim, where the antibiotic is ampicillin or kanamycin.

21. The genic construct according to any of the above claims, where the cleavage site is the cleavage target nucleotide sequence of the restriction enzyme Dra I or Bae I.

22. The genic construct according to any of the above claims, comprising SEQ ID NO: 1, SEQ ID NO: 2, SEQ ID NO: 3 or a biologically equivalent variant.

23. Use of the genic construct according to any of the above claims 1 to 20, for the amplification of an exogenous nucleotide sequence.

24. An oligonucleotide comprising the nucleotide sequence that contains between 65 and 130 nucleotides of the left extremity of the DNA of bacteriophage ϕ29.

25. The oligonucleotide according to the above claim, comprising the nucleotide sequence that contains between 67 and 72 nucleotides of the left extremity of the DNA of bacteriophage ϕ29.

26. The nucleotide according to any of the above claims, where the 5' extremity is phosphorylated.

27. The oligonucleotide according to any of the above three claims, comprising the target nucleotide sequence of a restriction enzyme or the nucleotide sequence resulting from cleavage with a restriction enzyme in position 3' with respect to the nucleotide sequence including between 65 and 130 nucleotides or between 67 and 72 nucleotides of the left extremity of the DNA of bacteriophage ϕ29.

28. Use of the oligonucleotide according to any of the claims 22 to 25, for constructing a recombinant linear nucleotide sequence in which an exogenous nucleotide sequence destined for amplification is flanked by nucleotide sequences comprising the sequence including between 65 and 130 nucleotides or between 67 and 72 nucleotides of the left extremity of the DNA of bacteriophage ϕ29.

29. A DNA amplification method comprising at least the following steps:
a) obtainment of a linear DNA molecule comprising the DNA sequence destined for amplification and flanked on both extremities by:
i) the sequence of the left replication origin of the DNA of bacteriophage ϕ29, the terminal extremity of this origin being located in the linear DNA extremity; or by
ii) the sequence of the right replication origin of the DNA of bacteriophage ϕ29, the terminal extremity of this origin being located in the linear DNA extremity; or by
iii) the sequence of the right replication origin of the DNA of bacteriophage ϕ29 on one side and of the left replication origin on the other - the terminal extremity of these origins being located in the two extremities of the linear DNA;
b) amplification of the linear DNA sequence obtained in step (a).

30. The method according to the above claim, where use is made of a DNA polymerase, a terminal protein (TP), a single strand DNA binding protein (SSB) and a double strand DNA binding protein (DBP) in the amplification of step (b).

31. The method according to either of the above two claims, where use is made of the proteins DNA polymerase, TP, p5 and p6 of bacteriophage ϕ29, or any bioequivalent variant of these proteins, in the amplification of step (b).

32. The method according to any of the above three claims, where the DNA sequence destined for amplification is between 500 and 100,000 bp in size.

33. The method according to any of the above four claims, where the linear DNA sequence obtained in step (a) presents phosphorylation of both 5' extremities.

34. The method according to any of the above 5 claims, where the sequence of the left replication origin of the DNA of bacteriophage ϕ29 contains between 65 and 200 nucleotides of the left extremity of the DNA of bacteriophage ϕ29.

35. The method according to the above claim, where the sequence of the left replication origin of the DNA of bacteriophage ϕ29 contains between 67 and 72 nucleotides of the left extremity of the DNA of bacteriophage ϕ29.

36. The method according to any of the above claims 29 to 33, where the sequence of the right replication origin of the DNA of bacteriophage ϕ29 contains between 125 and 250 nucleotides of the right extremity of the DNA of bacteriophage ϕ29.

37. The method according to the above claim, where the sequence of the right replication origin of the DNA of bacteriophage ϕ29 contains between 150 and 200 nucleotides of the right extremity of the DNA of bacteriophage ϕ29.

38. The method according to any of the above claims 29 to 33, where the sequence of the left replication origin of the DNA of bacteriophage ϕ29 contains between 65 and 200 nucleotides or between 67 and 72 nucleotides of the left extremity of the DNA of bacteriophage ϕ29, and the sequence of the right replication origin of the DNA of bacteriophage ϕ29 contains between 125 and 250 nucleotides or between 150 and 200 nucleotides of the right extremity of the DNA of bacteriophage ϕ29.

39. The method according to any of the above 10 claims, where step (b) is carried out at a temperature of under 30°C, preferably under 27°C, and even more preferably at a temperature of between 20-25°C.

40. The method according to any of the above 11 claims, where step (a) makes use of the genic construct according to any of the claims 1 to 22.

41. The method according to of the above claims 29 to 39, where step (a) makes use of the oligonucleotide according to any of the claims 24 to 27.

42. A kit for the amplification of an exogenous nucleotide sequence, consisting of at least a genic construct according to any of the above claims 1 to 22, at least an oligonucleotide according to any of the claims 24 to 27, or both.

43. The kit according to the above claim, where the genic construct comprises SEQ ID NO: 1, SEQ ID NO: 2 or SEQ ID NO: 3.

44. The kit according to either of the above two claims which moreover includes a DNA polymerase, a terminal protein (TP), a single strand DNA binding protein (SSB) and/or a double strand DNA binding protein (DBP).

45. The kit according to the above claim, where the DNA polymerase, the TP, the single strand DNA binding protein (SSB) and/or the double strand DNA binding protein (DBP) are the DNA polymerase, TP, p5 and/or p6 of bacteriophage ϕ29, or a bioequivalent variant of these proteins.

46. The kit according to any of the above four claims which moreover comprises at least one of the elements of the following list: deoxynucleotide triphosphates (dNTPs), (NH₄)₂SO₄, MgCl₂, dithiothreitol (DTT), glycerol, bovine serum albumin (BSA) and Tris-HCl buffer with a pH of between 6.5 and 8.

47. Use of the kit according to any of the above claims 42 to 46, for the amplification of an exogenous nucleotide sequence.
